# EUROPEAN PATENT APPLICATION

(11) **EP 4 006 552 A2**
(43) Date of publication of application: **01.06.2022**
(21) Application number: 20848228.1
(22) Date of filing: 30.07.2020
(51) Int. Cl.: G01N 33/569, G01N 33/53, C12Q 1/6869, G01N 30/68, G01N 30/88

(54) **KIT FOR PREDICTING OR DIAGNOSING NONALCOHOLIC FATTY LIVER DISEASE, AND METHOD FOR DIAGNOSING NONALCOHOLIC FATTY LIVER DISEASE**

(30) Priority: 30.07.2019 KR 20190092689; 14.07.2020 KR 20200087105
(71) Applicant: Kobiolabs, Inc., Seoul 08826 (KR)
(72) Inventor: KO, Gwang Pyo, Seoul 06732 (KR); KIM, Won, Seoul 06601 (KR); YOU, Hyun Ju, Incheon 21545 (KR); LEE, Giljae, Seoul 08862 (KR)
(74) Representative: Grünecker Patent- und Rechtsanwälte PartG mbB
(86) International application number: PCT/KR2020/010092
(87) International publication number: WO 2021/020920

(57) **Abstract**

The present invention relates to a kit for predicting or diagnosing the degree of risk of disease, a method for providing information for predicting or diagnosing the degree of risk of disease, a method for screening a therapeutic agent of disease, and a pharmaceutical composition for prevention or treatment of disease, for nonalcoholic fatty liver disease. Specifically, through the kit for predicting or diagnosing of the present invention, the degree of risk of nonalcoholic fatty liver disease can be effectively predicted or diagnosed, and in particular, the predictive value and significance of information provision in non-obese subjects are excellent. Therefore, by providing effective information on nonalcoholic fatty liver disease through this, it can be effectively used to prevent or treat the corresponding disease. In addition, the pharmaceutical composition for prevention or treatment of the present invention may be effectively used for treatment of nonalcoholic fatty liver disease.

## Description

### [TECHNICAL FIELD]

The present invention relates to a kit for predicting or diagnosing nonalcoholic fatty liver disease, and a method for diagnosing thereof.

### [BACKGROUND ART]

Nonalcoholic fatty liver disease (NAFLD) is characterized by liver disease of metabolic disorders ranging from simple steatosis, to nonalcoholic steatohepatitis (NASH) which is an aggressive tissue form that ultimately leads to advanced fibrosis and cirrhosis. The global prevalence of NAFLD is estimated to be 24-30% in most epidemiological studies, and is increasing in parallel with obesity and metabolic syndrome. Although NAFLD is commonly associated with obesity, clinical symptoms and pathological severity similar to those observed in obese NAFLD patients may occur in non-obese subjects. Without considering the cut-off of the different body mass index (BMI) defining obesity (Asian ≥25, other races ≥30), it is consistently reported that 3-30% of the non-obese population has NAFLD in both the West and the East. Although visceral fat, food composition and genetic factors may be associated with non-obese NAFLD, additional studies considering other environmental factors are needed to elucidate the pathogenesis of non-obese NAFLD.

Recently, increased interests have focused on identifying and understanding specific roles of the gut microbiota in various metabolic diseases. Gut dysbiosis, which refers to abnormal changes in the gut microbiota compared to the normal microbiota, is associated with a decrease in bacteria producing beneficial short chain fatty acid (SCFA), changes in bile acid composition, activation of immune response against lipopolysaccharide (LPS), an increase of ethanol production by hyperplasia of ethanol producing bacteria, and conversion of phosphatidylcholine into choline and trimethylamine. Changes in the gut microbiome that affects the gut-liver axis contribute to the progression of chronic liver disease such as NAFLD and cirrhosis and advanced fibrosis.

Boursier et al. compared microbiome changes between patients with mild and severs fibrosis, and observed significant intestinal bacterial imbalance and functional changes in patients with severe fibrosis (Non-patent Document 1). Loomba et al. used metagenomic data to identify 37 bacteria that were significantly enriched or significantly reduced in NAFLD patients with advanced fibrosis, and proposed a microbiome-based biomarker to predict fibrosis (Non-patent Document 2). Bajaj et al. defined the gut microbiome profile during the progression of cirrhosis (Non-patent Document 3). A Chinese cohort study observed changes in the gut microbiome of cirrhosis patients (Non-patent Document 4). However, the microbial taxa associated with disease severity and fibrosis stage were not consistent with previous NAFLD studies. This discrepancy may be due to the influence of regional differences (Non-patent Document 5). However, differences in basic BMI status may explain these inconsistent results. Moreover, specific changes in gut microbiome and related metabolites in the non-obese NAFLD group were rarely defined.

Therefore, there is a need for a method for preventing, treating and diagnosing non-obese NAFLD, which determines the histological severity of NAFLD, well-characterizes the gut microbiome changes, and is effective.

### [DISCLOSURE]

### [TECHNICAL PROBLEM]

The present invention is to solve the above problem, and its purpose is to provide a detection marker of nonalcoholic fatty liver disease comprising one or more of detection markers selected from the group consisting of a microbial biomarker, bile acid and components thereof, and intestinal short chain fatty acid, a kit for predicting or diagnosing a degree of risk of nonalcoholic fatty liver disease using the detection marker, a method for predicting or diagnosing, or a method for providing information for predicting or diagnosing the degree of risk of nonalcoholic fatty liver disease using the detection marker, and a method for screening a therapeutic agent of nonalcoholic fatty liver disease.

### [TECHNICAL SOLUTION]

In order to achieve the above purpose, the present invention provides a detection marker of nonalcoholic fatty liver disease and a kit for predicting or diagnosing a degree of risk of nonalcoholic fatty liver disease comprising one or more detection means detecting the detection marker.

The kit may be used for predicting or diagnosing a degree of risk of nonalcoholic fatty liver disease by comprising the aforementioned means of detecting a specific subject and specifying the amount, activity, population, and the like of the specific detection subject, or comparing results with other detection subject.

In the present invention, diagnosis comprises confirming the presence or absence of disease, degree of risk of disease, state of disease and prognosis of disease, and comprises all types of analysis used to derive disease state and decision.

In an embodiment of the present invention, the nonalcoholic fatty liver disease may be nonalcoholic fatty liver, nonalcoholic steatohepatitis or cirrhosis.

In an embodiment of the present invention, predicting the degree of risk of the disease may predict the severity of fibrosis. The severity of fibrosis may include F=0 to F=4, and F=0 means no liver fibrosis; F=1 means mild liver fibrosis; F=2 means significant liver fibrosis; F=3 means advanced liver fibrosis; and F=4 means cirrhosis.

In an embodiment of the present invention, the kit may be for a non-obese patient, for example, a non-obese patient with BMI of 25 kg/m² or less.

The detection marker of nonalcoholic fatty liver disease may comprise one or more kinds among microbial biomarkers, total bile acid and components, and intestinal short chain fatty acid.

In an embodiment of the present invention, the kit may comprise a detection means capable of detecting one or more of detection markers selected from the following:
(a) one or more detection means respectively detecting one or more selected from the group consisting of microbial biomarkers of nonalcoholic fatty liver disease;
(b) one or more detection means respectively detecting one or more selected from the group consisting of total bile acid and components of bile acid; and
(c) one or more detection means respectively detecting one or more selected from the group consisting of intestinal short chain fatty acids.

The kit may comprise for example, (a); (b); (c); (a) and (b); (a) and (c); (b) and (c); or (a), (b), and (c).

In the present specification, the total bile acid and components of bile acid, the short chain fatty acid, and the like are used as a meaning comprising all metabolites thereof.

The microbial biomarkers of nonalcoholic fatty liver disease, at the Family level, may be one or more selected from the group consisting of Enterobacteriaceae, Veillonellaceae, Rikenellaceae, Fusobacteriaceae, Ruminococcaceae, Lachnospiraceae, Actinomycetaceae, Desulfovibrioceae, and Desulfovibrionaceaeat. For example, it may be one or more selected from the group consisting of Enterobacteriaceae, Veillonellaceae, Ruminococcaceae, Lachnospiraceae and Actinomycetaceae, one or more selected from the group consisting of Enterobacteriaceae, Veillonellaceae, Lachnospiraceae and Ruminococcaceae, or one or more selected from the group consisting of Enterobacteriaceae, Veillonellaceae, and Ruminococcaceae.

One example of the Enterobacteriaceae microorganism may be one or more of Citrobacter and Klebsiella, one example of the Veillonellaceae microorganism may be one or more of Veillonella and Megamonas, one example of the Fusobacteriaceae microorganism may be Fusobacterium, one example of the Desulfovibrionaceae microorganism may be Desulfovibrio, the Ruminococcaceae microorganism may be one or more of Ruminococcus, Faecalibacterium and Oscillospira, the Lachnospiraceae microorganism may be one or more of Coprococcus and Lachnospira, and the Actinomycetaceae microorganism may be actinomyces.

As one example, the (a) may be one or more of detection means each capable of detecting one or more selected from the group consisting of Enterobacteriaceae, Veillonellaceae, Lachnospiraceae and Ruminococcaceae.

The microbial biomarker according to the present invention, at the genus level, may be one or more kinds selected from the group consisting of Citrobacter, Klebsiella, Ruminococcus, Faecalibacterium, Oscillospira, Coprococcus, and Lachnospira, and specifically, it may be one or more kinds selected from the group consisting of Ruminococcus, Faecalibacterium, Oscillospira, Coprococcus, Veillonella, Megamonas, and Lachnospira, and more specifically, it may be one or more kinds selected from the group consisting of Ruminococcus, Faecalibacterium, Oscillospira, Coprococcus, and Lachnospira, or one or more kinds selected from the group consisting of Veillonella and Megamona.

The total bile acid and components of bile acid may be one or more selected from the group consisting of primary bile acid comprising total bile acid, cholic acid and chenodeoxycholic acid; and secondary bile acid comprising ursodeoxycholic acid, lithocholic acid and deoxycholic acid.

As one example, the (b) may be one or more of detection means each capable of detecting one or more selected from the group consisting of total bile acid, cholic acid, chenodeoxycholic acid, ursodeoxycholic acid, lithocholic acid and deoxycholic acid.

As one example, the (c) may be one or more of detection means each capable of detecting one or more selected from the group consisting of acetate, propionate and butyrate.

In an embodiment of the present invention, the kit may comprise one or more selected from combinations of (a) to (h) as follows:
detection means of the (a),
detection means of the (b),
detection means of the (c),
(d) one or more detection means each capable of detecting one or more selected from the group consisting of Enterobacteriaceae, Veillonellaceae, Lachnospiraceae, Ruminococcaceae, cholic acid, chenodeoxycholic acid, ursodeoxycholic acid and propionate;
(e) one or more detection means each capable of detecting one or more selected from the group consisting of Ruminococcus, Faecalibacterium, Oscillospira, Coprococcus, Lachnospira, total bile acid, cholic acid, chenodeoxycholic acid, ursodeoxycholic acid, lithocholic acid and deoxycholic acid;
(f) one or more detection means each capable of detecting one or more selected from the group consisting of Ruminococcus, Faecalibacterium, Oscillospira, Coprococcus, Lachnospira and fecal propionate;
(g) one or more detection means each capable of detecting one or more selected from the group consisting of Veillonella, Megamonas, total bile acid, cholic acid, chenodeoxycholic acid, ursodeoxycholic acid, lithocholic acid and deoxycholic acid; and
(h) one or more detection means each capable of detecting one or more selected from the group consisting of Veillonella, Megamonas and fecal propionate.

The detection means of the (a) may be, for example, one or more detection means each capable of detecting one or more selected from the group consisting of Enterobacteriaceae, Veillonellaceae, Lachnospiraceae and Ruminococcaceae.

The detection means of the (b) may be, for example, one or more detection means each capable of detecting one or more selected from the group consisting of total bile acid, cholic acid, chenodeoxycholic acid, ursodeoxycholic acid, lithocholic acid and deoxycholic acid.

The detection means of the (c) may be, for example, one or more detection means each capable of detecting one or more selected from the group consisting of short chain fatty acid, acetate, propionate and butyrate.

In an embodiment of the present invention, the kit may comprise one or more selected from combinations of (i) to (k) as follows:
(i) one or more detection means each capable of detecting one or more selected from the group consisting of Enterobacteriaceae, Veillonellaceae, Ruminococcaceae, Citrobacter, Klebsiella, Veillonella, Megamonas, Ruminococcus, Faecalibacterium and Oscillospira;
(j) one or more detection means each capable of detecting one or more selected from the group consisting of cholic acid, chenodeoxycholic acid, ursodeoxycholic acid and metabolites thereof; and
(k) one or more detection means each capable of detecting one or more selected from the group consisting of intestinal short chain fatty acids and propionate.

As one example, the detection marker of nonalcoholic fatty liver disease according to the present invention may comprise one or more kinds selected from the group consisting of Enterobacteriaceae, Veillonellaceae, Rikenellaceae, Fusobacteriaceae, Ruminococcaceae, Lachnospiraceae, Actinomycetaceae, Desulfovibrioceae, Desulfovibrionaceae, Citrobacter, Klebsiella, Veillonella, Megamonas, Fusobacterium, Ruminococcus, Faecalibacterium, Oscillospira, Coprococcus, Lachnospira, Actinomyces, Desulfovibrio, total bile acid, cholic acid, chenodeoxycholic acid, ursodeoxycholic acid, lithocholic acid, deoxycholic acid, short chain fatty acid, acetate, propionate and butyrate.

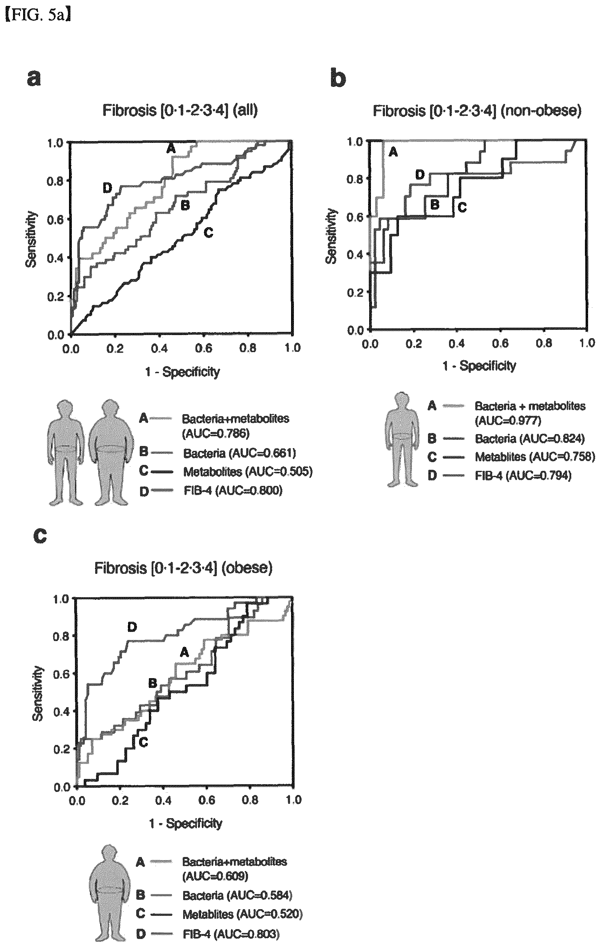

As one specific example, the detection marker of nonalcoholic fatty liver disease according to the present invention may comprise Enterobacteriaceae, Veillonellaceae, and Ruminococcaceae. According to the Examples of the present application, in case of using the three bacterial markers in combination, non-alcoholic fatty liver can be predicted with high accuracy of AUROC 0.8 or higher in a non-obese subject (FIG. 5a).

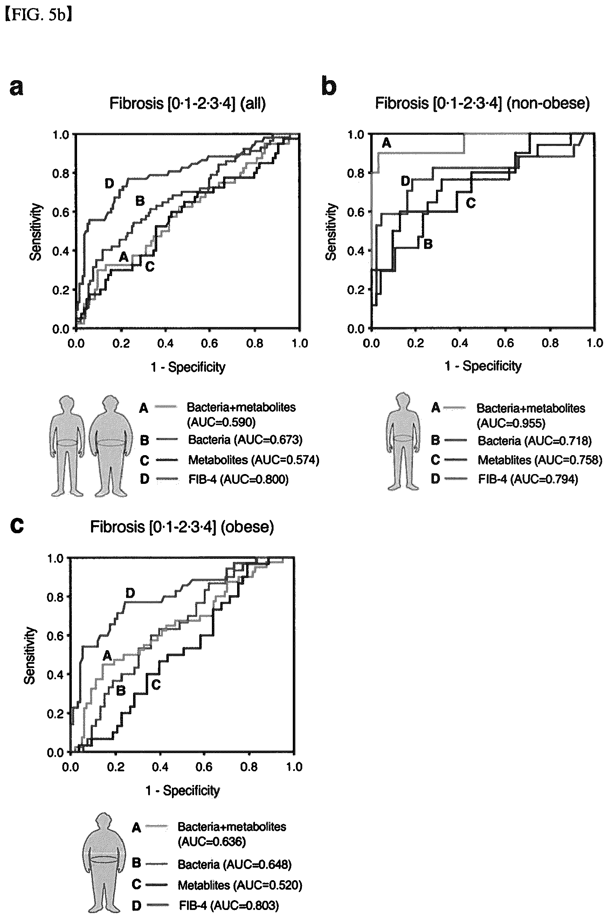

As one specific example, the detection marker of nonalcoholic fatty liver disease according to the present invention may comprise Megamonas and Ruminococcus. According to the Examples of the present application, in case of using the two bacterial markers in combination, non-alcoholic fatty liver could be predicted with high accuracy of AUROC 0.7 or higher in a non-obese subject (FIG. 5b).

As one specific example, the detection marker of nonalcoholic fatty liver disease according to the present invention may comprise cholic acid, chenodeoxycholic acid, ursodeoxycholic acid, and propionate. According to the Examples of the present application, in case of using 4 metabolite markers in combination, non-alcoholic fatty liver could be predicted with high accuracy of AUROC 0.7 or higher in a non-obese subject.

As one specific example, the detection marker of nonalcoholic fatty liver disease according to the present invention may comprise Enterobacteriaceae, Veillonellaceae, Ruminococcaceae, cholic acid, chenodeoxycholic acid, ursodeoxycholic acid, and propionate. According to the Examples of the present application, in case of using the 3 bacterial markers and 4 metabolite markers in combination, non-alcoholic fatty liver could be predicted with high accuracy of AUROC 0.9 or higher in a non-obese subject. Otherwise, the detection marker of nonalcoholic fatty liver disease according to the present invention may comprise Megamonas, Ruminococcus, cholic acid, chenodeoxycholic acid, ursodeoxycholic acid, and propionate. According to the Examples of the present application, in case of using the 2 bacterial markers and 4 metabolite markers in combination, non-alcoholic fatty liver could be predicted with high accuracy of AUROC 0.9 or higher in a non-obese subject. This was significantly higher accuracy than the biomarker of non-alcoholic fatty liver used conventionally, and thus, it could be seen that the biomarker for predicting non-alcoholic fatty liver according to the present invention could predict non-alcoholic fatty liver accurately, and in particular, it could predict non-alcoholic fatty liver of a non-obese subject more accurately.

The present invention provides a method for predicting or diagnosing, or a method for providing information for predicting or diagnosing a degree of risk of nonalcoholic fatty liver disease comprising detecting one or more of detection markers selected from the group consisting of (a) one or more detection markers selected from the group consisting of microbial biomarkers of nonalcoholic fatty liver disease; (b) one or more detection markers selected from the group consisting of total bile acid and components of bile acid; and (c) one or more detection markers selected from the group consisting of intestinal short chain fatty acids.

In an embodiment of the present invention, the nonalcoholic fatty liver disease may be nonalcoholic fatty liver, nonalcoholic steatohepatitis or cirrhosis. In an embodiment of the present invention, predicting the degree of risk may be predicting the severity of fibrosis. In an embodiment of the present invention, the method for diagnosing or method for providing information for diagnosing may be for a non-obese patient with BMI<25 kg/m².

In an embodiment of the present invention, the method may comprise one or more steps selected from the following:
(i) measuring abundance of microbial biomarkers as one or more detection markers selected from the group consisting of microbial biomarkers of nonalcoholic fatty liver disease;
(ii) measuring the content in feces of one or more detection markers selected from the group consisting of total bile acid and components of bile acid; and
(iii) measuring the content in feces of one or more detection markers selected from the group consisting of intestinal short chain fatty acid, for example, acetate, propionate and butyrate.

In an embodiment of the present invention, the method may comprise comparing the detected values of a subject individual, with a reference value of a healthy individual corresponding thereto, for detection markers (a) to (c), (a) to (h), (i) to (k), or (a) to (k) which can be comprised in the kit.

In an embodiment of the present invention, the method may comprise determining that the severity of fibrosis is high, the detected value of the subject individual compared to the reference value of the healthy individual is increased or decreased according to an increase or decrease of the following detection markers, as the result of comparing the detected values of the subject and reference value of the healthy individual corresponding thereto:
(a) with respect to one or more detection markers selected from the group consisting of microbial biomarkers of nonalcoholic fatty liver disease, the abundance of Enterobacteriaceae is increased, the abundance of Veillonellaceae is increased, or the abundance of Ruminococcaceae is decreased,
(b) with respect to one or more detection markers selected from the group consisting of total bile acid and components of bile acid, the content of cholic acid in feces is increased, the content of chenodeoxycholic acid in feces is increased, or the content of ursodeoxycholic acid in feces is increased, or
(c) with respect to one or more detection markers selected from the group consisting of intestinal short chain fatty acids, for example, in case that the content of one or more selected from the group consisting of acetate, propionate and butyrate in feces is increased, as one example, in case that the content of propionate in feces is increased, it may comprise determining that the severity of fibrosis is high.

The present invention provides a method for screening a therapeutic agent for nonalcoholic fatty liver disease comprising the following steps:
(1) administering a test substance to an experimental animal having nonalcoholic fatty liver disease;
(2) measuring one or more detection markers selected from the group consisting of (a) one or more detection markers selected from the group consisting of microbial biomarkers of nonalcoholic fatty liver disease; (b) one or more detection markers selected from the group consisting of total bile acid and components of bile acid; and (c) one or more detection markers selected from the group consisting of intestinal short chain fatty acids, in the experimental animal untreated with the test substance and the experimental animal treated with the test substance; and
(3) comparing the measured results in a control group untreated with the test substance and an experimental group administered with the test substance.

In an embodiment of the present invention, the nonalcoholic fatty liver disease may be nonalcoholic fatty liver, nonalcoholic steatohepatitis or cirrhosis.

In an embodiment of the present invention, the experimental animal of the step (1) may have one or more characteristics of the following (1) to (5):
(1) A condition in which the blood ALT concentration is increased, for example, a condition in which it is over 1 time, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.1 times or more, 2.2 times or more, 2.3 times or more, 2.4 times or more, 2.5 times or more, 2.6 times or more, 2.7 times or more, 2.8 times or more, 2.9 times or more, 3 times or more, 3.5 times or more, 4 times or more, 4.5 times or more, 5 times or more, 5.5 times or more, 6 times or more, 6.5 times or more, 7 times or more, 7.5 times or more, 8 times or more, 8.5 times or more, 9 times or more, 9.5 times or more, or 10 times or more, of the blood ALT concentration of a normal control group.
(2) A condition in which the blood AST concentration is increased, for example, a condition in which it is over 1 time, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.1 times or more, 2.2 times or more, 2.3 times or more, 2.4 times or more, 2.5 times or more, 2.6 times or more, 2.7 times or more, 2.8 times or more, 2.9 times or more, 3 times or more, 3.5 times or more, 4 times or more, 4.5 times or more, 5 times or more, 5.5 times or more, 6 times or more, 6.5 times or more, 7 times or more, 7.5 times or more, 8 times or more, 8.5 times or more, 9 times or more, 9.5 times or more, or 10 times or more, of the blood AST concentration of a normal control group.
(3) A condition in which the secondary bile acid concentration in cecum is decreased, for example, a condition in which it is less than 1 time, 0.9 times or less, 0.8 times or less, 0.7 times or less, 0.6 times or less, 0.5 times or less, 0.4 times or less, 0.3 times or less, 0.2 times or less, or 0.1 time or less, of the secondary bile acid concentration in cecum of a normal control group.
(4) A condition in which the fibrosis marker gene expression is increased, for example, a condition in which it is overexpressed more than 1 time, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.1 times or more, 2.2 times or more, 2.3 times or more, 2.4 times or more, 2.5 times or more, 2.6 times or more, 2.7 times or more, 2.8 times or more, 2.9 times or more, 3 times or more, 3.5 times or more, 4 times or more, 4.5 times or more, 5 times or more, 5.5 times or more, 6 times or more, 6.5 times or more, 7 times or more, 7.5 times or more, 8 times or more, 8.5 times or more, 9 times or more, 9.5 times or more, 10 times or more, 11 times or more, 12 times or more, 13 times or more, 14 times or more, 15 times or more, 16 times or more, 17 times or more, 18 times or more, 19 times or more, or 20 times or more, of the fibrosis marker gene expression of a normal control group. The fibrosis marker gene may be one or more kinds selected from the group consisting of Col1a1, Timp1, and α-SMA.
(5) A condition in which the liver weight ratio to body weight is increased, for example, a condition in which it is over 1 time, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.1 times or more, 2.2 times or more, 2.3 times or more, 2.4 times or more, 2.5 times or more, 2.6 times or more, 2.7 times or more, 2.8 times or more, 2.9 times or more, or 3 times or more, of the liver weight ratio to body weight of a normal control group.

In an embodiment of the present invention, the test substance may comprise a candidate substance of a therapeutic agent for nonalcoholic fatty liver disease, and for example, it may be one or more selected from the group consisting of peptide, protein, nonpeptide compound, active compound, fermented product, cell extract, plant extract, animal tissue extract and plasma.

In an embodiment of the present invention, the method may comprise one or more steps selected in the following:
measuring the content in feces of one or more detection markers selected from the group consisting of
(i) measuring abundance of microbial biomarkers as one or more detection markers selected from the group consisting of microbial biomarkers of nonalcoholic fatty liver disease;
(ii) measuring the content in feces of one or more detection markers selected from the group consisting of total bile acid and components of bile acid; and
(iii) measuring the content in feces of one or more detection markers selected from the group consisting of intestinal short chain fatty acid, for example, acetate, propionate and butyrate.

In an embodiment of the present invention, the method may comprise comparing detection values of an experimental group administered with a test substance and a control group not administered with the test substance for an experimental animal having nonalcoholic fatty liver disease, for detection markers (a) to (c), (a) to (h), (i) to (k), or (a) to (k).

In an embodiment of the present invention, the method may comprise selecting a test substance as a therapeutic agent of nonalcoholic fatty liver disease, according to an increase or decrease result for each detection marker of the detected value of the experimental group compared to a reference value of the control group, as the result of comparing the detected values of an experimental group and the detected value of a control group:
(a) with respect to one or more detection markers selected from the group consisting of microbial biomarkers of nonalcoholic fatty liver disease, the abundance of Enterobacteriaceae is increased, the abundance of Veillonellaceae is increased, or the abundance of Ruminococcaceae is decreased,
(b) with respect to one or more detection markers selected from the group consisting of total bile acid and components of bile acid, the content in feces of cholic acid is increased, the content in feces of chenodeoxycholic acid is increased, or the content in feces of ursodeoxycholic acid is increased, or
(c) with respect to one or more detection markers selected from the group consisting of intestinal short chain fatty acids, for example, in case that the content in feces of one or more selected from the group consisting of acetate, propionate and butyrate is increased, or as one example, the content in feces of propionate is increased, a step of determining that the severity of fibrosis is high may be comprised.

In an embodiment of the present invention, the method may comprise selecting a test substance in which the abundance of Enterobacteriaceae is decreased, the abundance of Veillonellaceae is decreased, the abundance of Ruminococcaceae is increased, the content in feces of cholic acid is decreased, the content in feces of chenodeoxycholic acid is decreased, the content in feces of ursodeoxycholic acid is decreased, or the content in feces of propionate is decreased, compared to the control group untreated with the test substance.

The method for predicting a degree of risk of nonalcoholic fatty liver disease, the method for providing information for prediction, the method for diagnosis, or the method for providing information for diagnosis, according to one example of the present invention may further comprise administering a therapeutic agent of nonalcoholic fatty liver disease to a subject.

Other embodiment of the present invention relates to a method for treatment of nonalcoholic fatty liver disease, comprising administering a therapeutic agent of nonalcoholic fatty liver disease to a subject determined as having risk of nonalcoholic fatty liver disease by the kit for predicting or diagnosing a degree of risk of nonalcoholic fatty liver disease, the method for predicting a degree of risk of nonalcoholic fatty liver disease, the method for providing information for prediction, the method for diagnosis, or the method for providing information for diagnosis, according to the present invention.

### [ADVANTAGEOUS EFFECTS]

The degree of risk of nonalcoholic fatty liver disease can be effectively predicted or diagnosed using the kit for predicting or diagnosing of the present invention, and in particular, the predictability and significance of information provision in non-obese subjects are excellent. Therefore, it can be effectively used to prevent or treat the corresponding disease by providing effective information on nonalcoholic fatty liver disease through this.

### [BRIEF DESCRIPTION OF THE DRAWINGS]

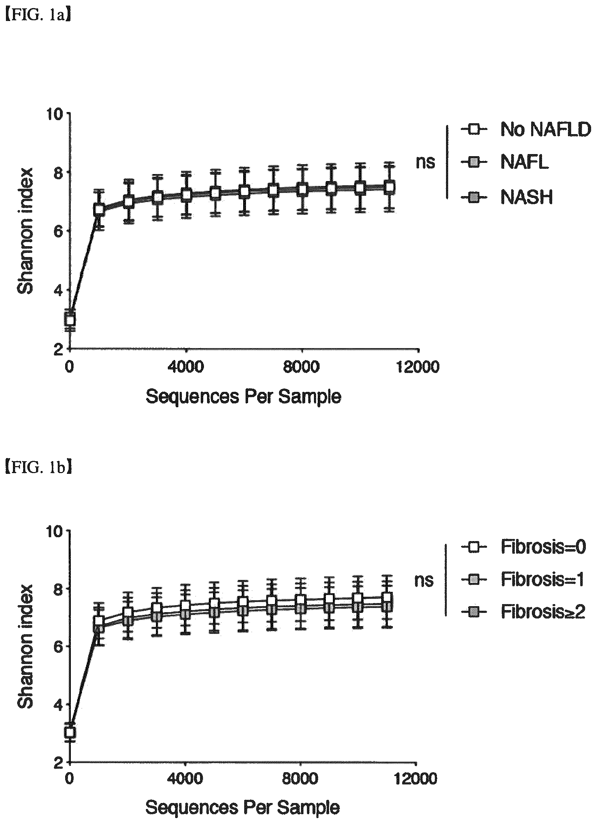

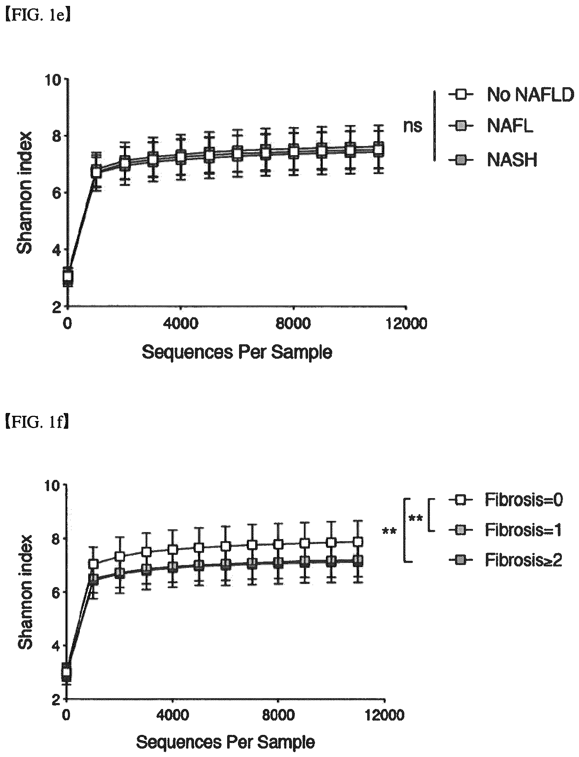

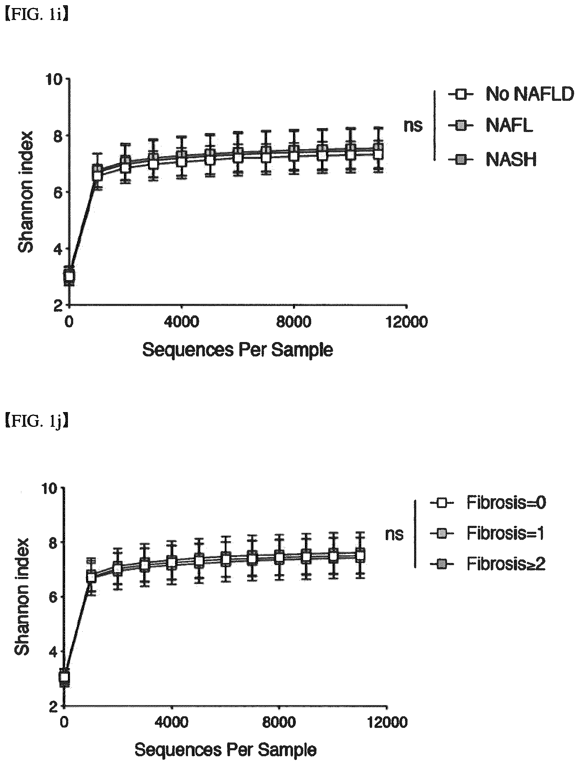

FIG. 1a to 11 are results of comparing the diversities of the gut microbiome. Values dividing the alpha and beta diversity by histological spectrum of NAFLD or fibrosis severity of all subjects are shown in FIG. 1a to 1d, of non-obese subjects are shown in FIG. 1e to 1h, and of obese subjects are shown in FIG. 1i to FIG. 11. Rarefaction curves were generated using the Shannon index with 12,000 sequences per sample. Statistical analysis was performed using nonparametric Kruskal-Wallis test. NMDS plots were generated using relative OTU abundance data according to Bray-Curtis distance, and statistical significance was determined using Adonis analysis. ^{∗∗}*P*<0.01

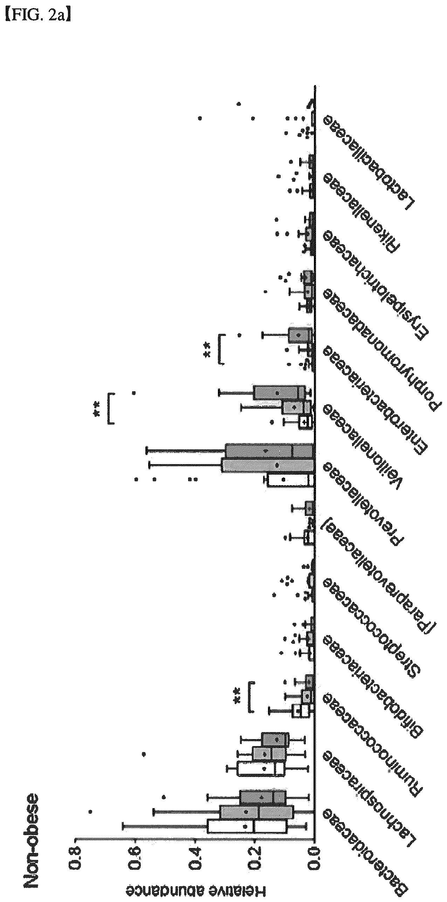

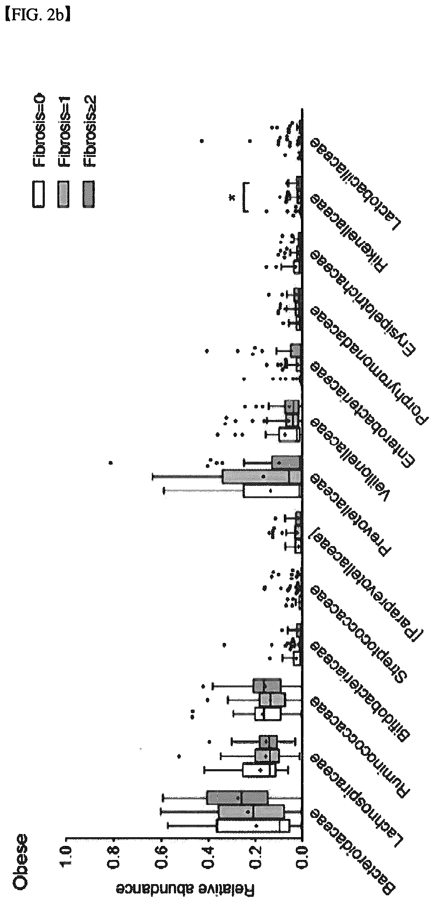

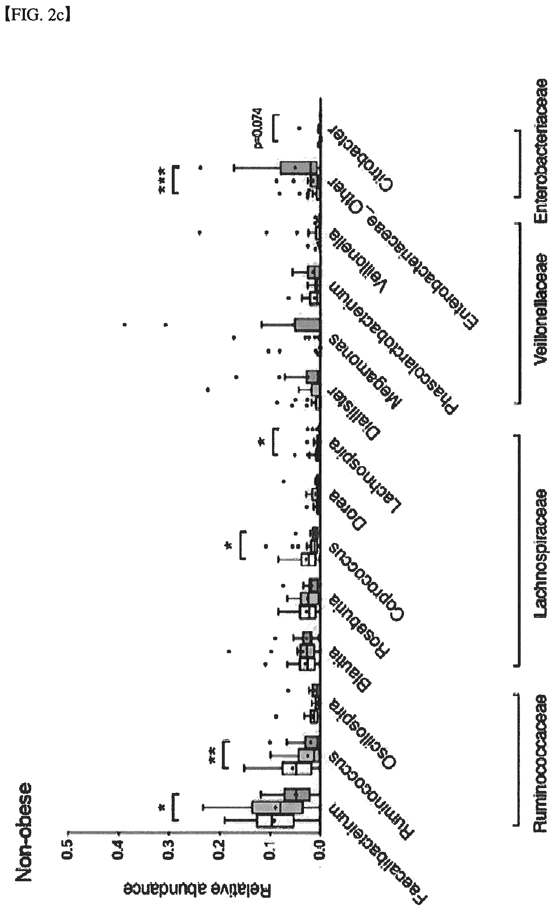

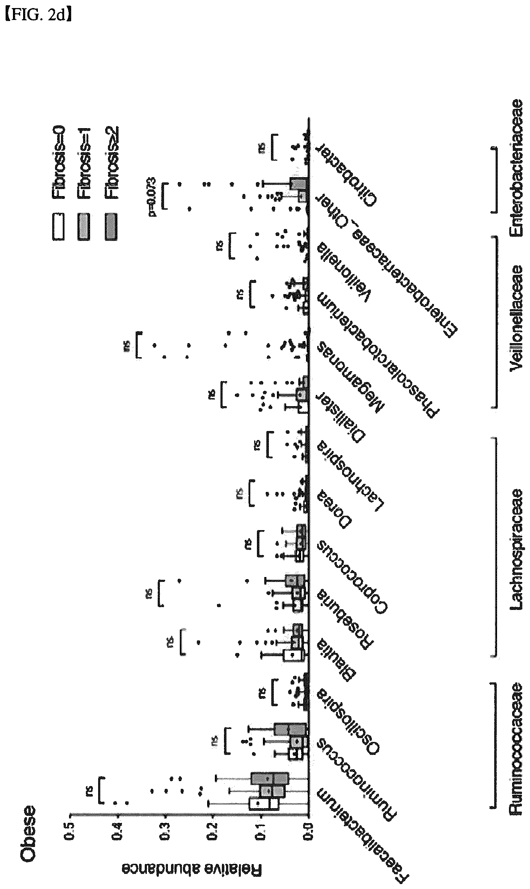

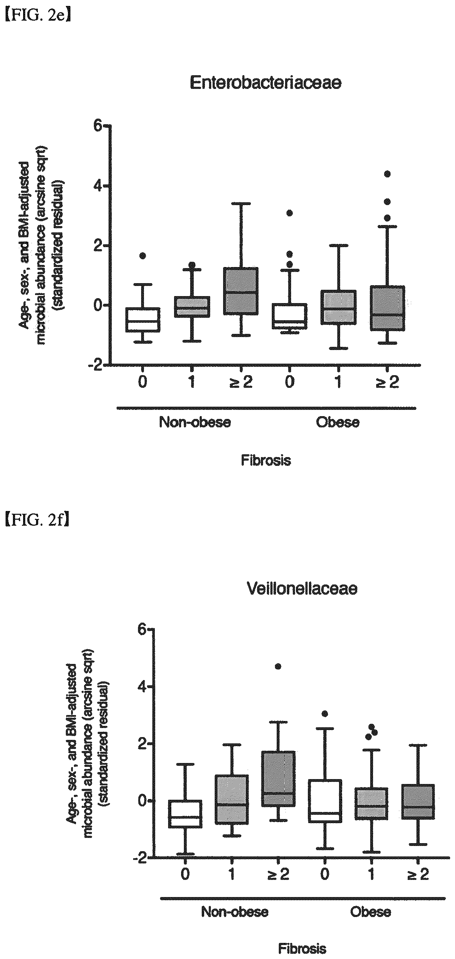

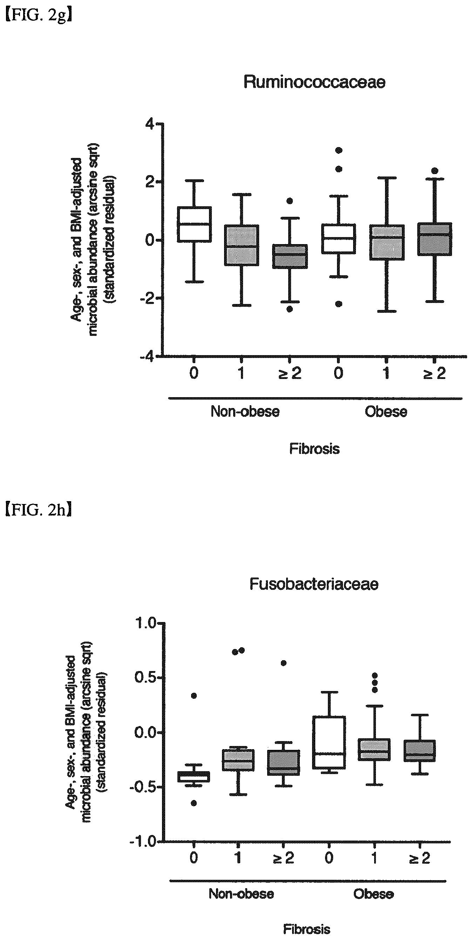

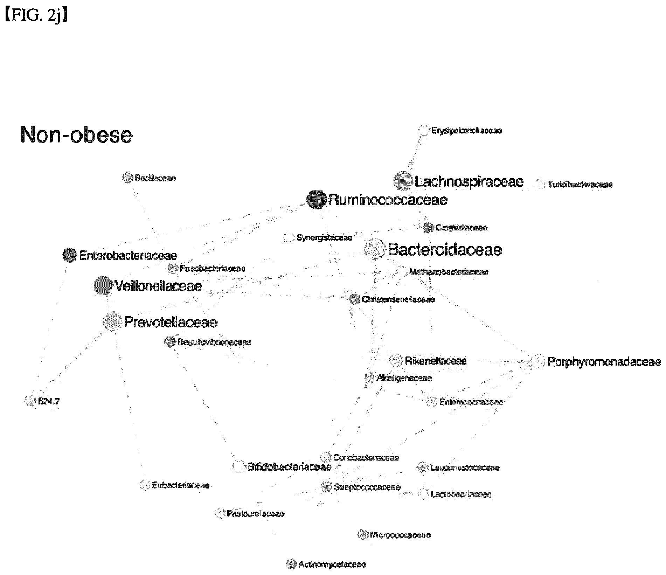

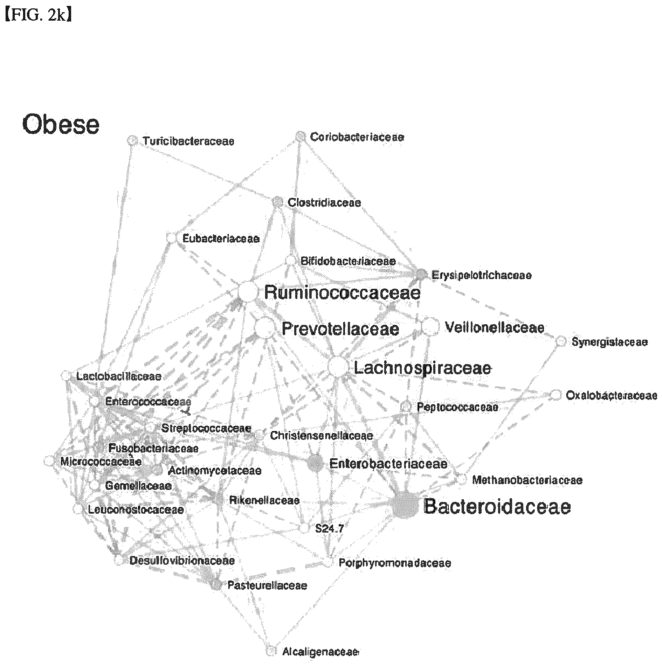

FIG. 2a to 2d are univariate analysis results for differences in specific microbial taxa according to the severity of fibrosis. For clarity, 13 family- and 14 genus-level taxa are shown along with their upper relative abundance. The box plot shows the interquartile range (IQR) between the first and third quartiles with Tukey whiskers. The color in the box indicates the severity of fibrosis. For statistical significance, a nonparametric Kruskal-Wallis test was used. ^{∗}*P*<0.05, ^{∗∗}*P*<0.01, ^{∗∗∗}*P*<0.001
FIG. 2e to 2h are multivariate analysis results for differences in specific microbial taxa according to the severity of fibrosis. Arcsine root-modified abundance of four bacteria was regressed for age, gender and BMI according to the severity of fibrosis, and the standard residual was indicated as a box plot. ^{∗}*P*<0.05, ^{∗∗}*P*<0.01, ^{∗∗∗}*P*<0.001

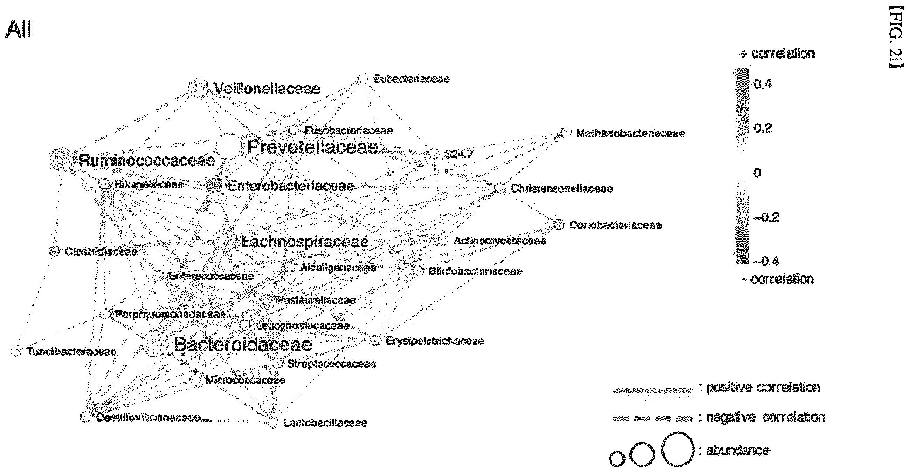

FIG. 2i to 2k are results of co-expression analysis of specific gut microbiota elements in total, non-obese and obese subjects. Solid lines (orange) and dotted lines (grey) indicate positive and negative correlations, respectively. The size of the node indicates the relative amount of bacteria, and the color indicates the degree of correlation according to the severity of fibrosis.

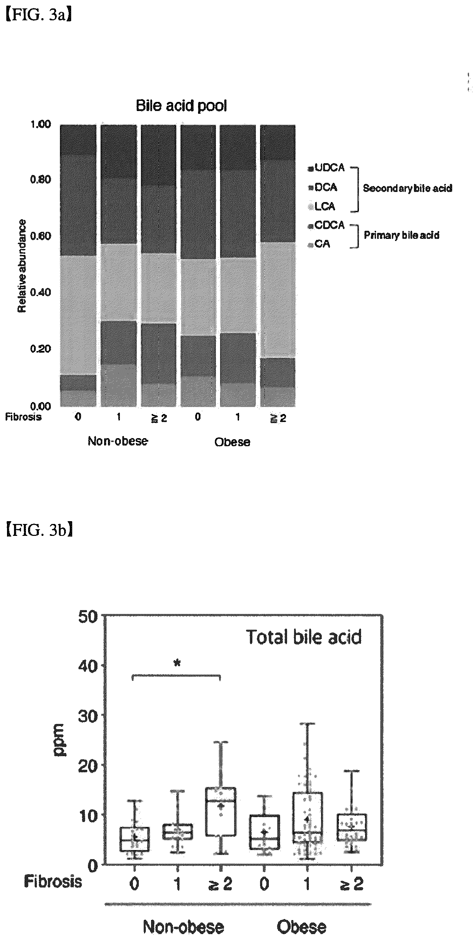

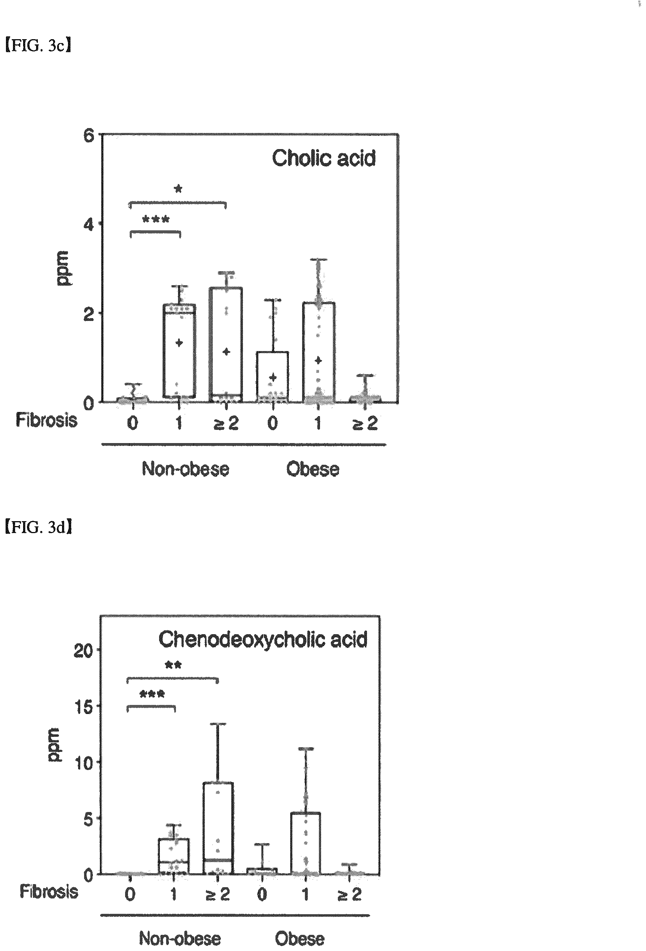

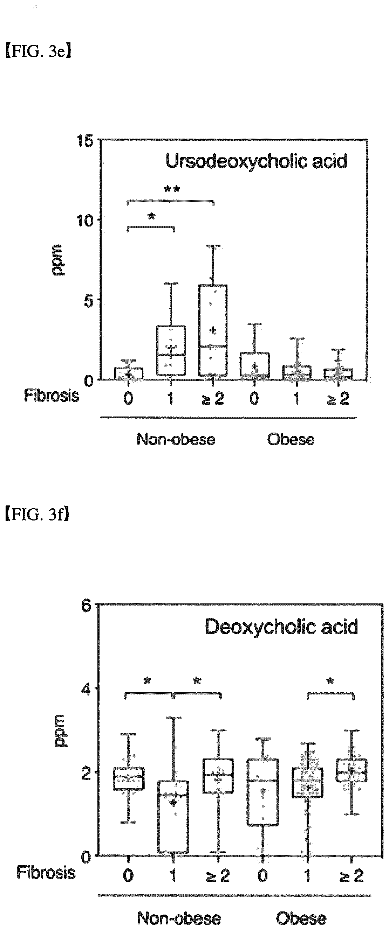

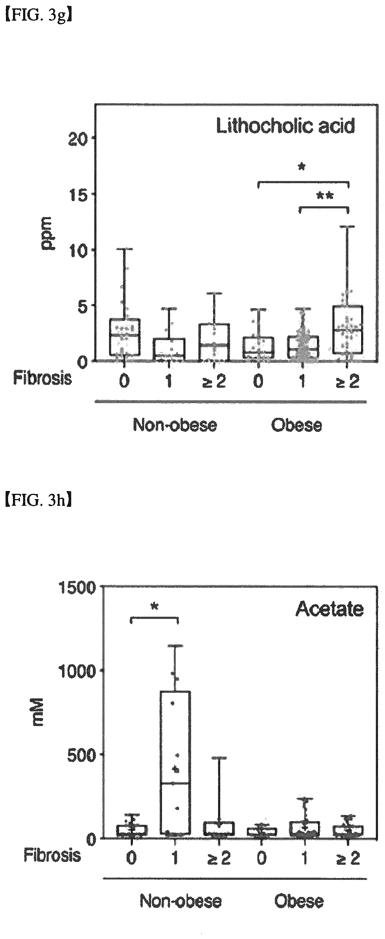

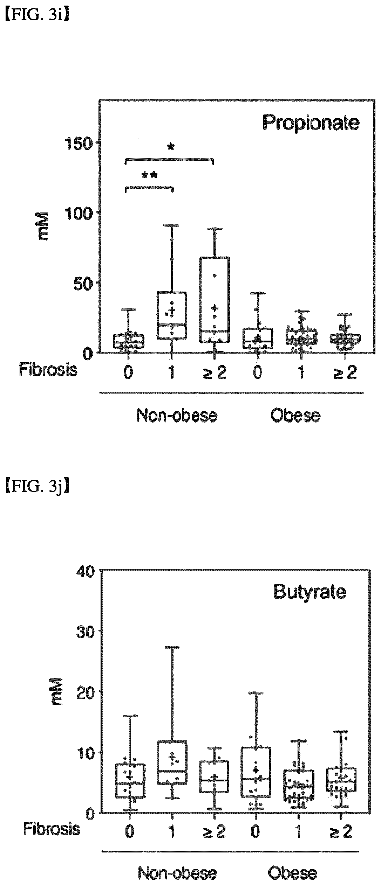

Fig. 3a to 3j are evaluation results of fecal metabolites mainly related to the gut microbiota. FIG. 3a is the bile acid profile result in various clinical environments, and stacked plots were generated using the average abundance of five bile acids. In FIG. 3b to 3g, the box plots show stratified fecal bile acid levels according to fibrosis severity and obesity status. The concentrations of the five fecal bile acids were stratified by fibrosis severity and obesity status. In FIG. 3h to FIG. 3j, the box plots show the most abundant fecal SCFA (acetate, propionate and butyrate) stratified by fibrosis severity and obesity status. The interquartile ranges (IQRs) between the first and third quartiles are described as Tukey whiskers. For statistical significance, a nonparametric Kruskal-Wallis test was used. ^{∗}*P*<0.05, ^{∗∗}*P*<0.01, ^{∗∗∗}*P*<0.001

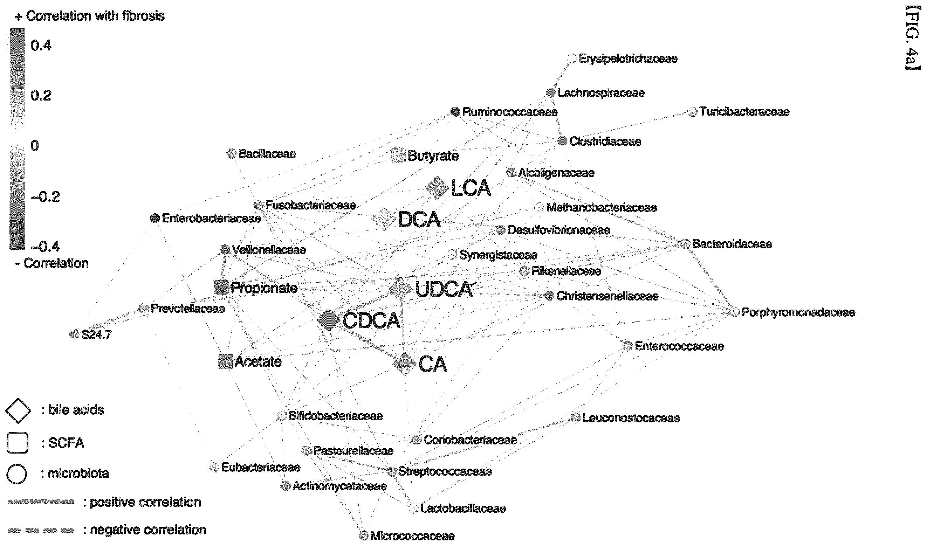

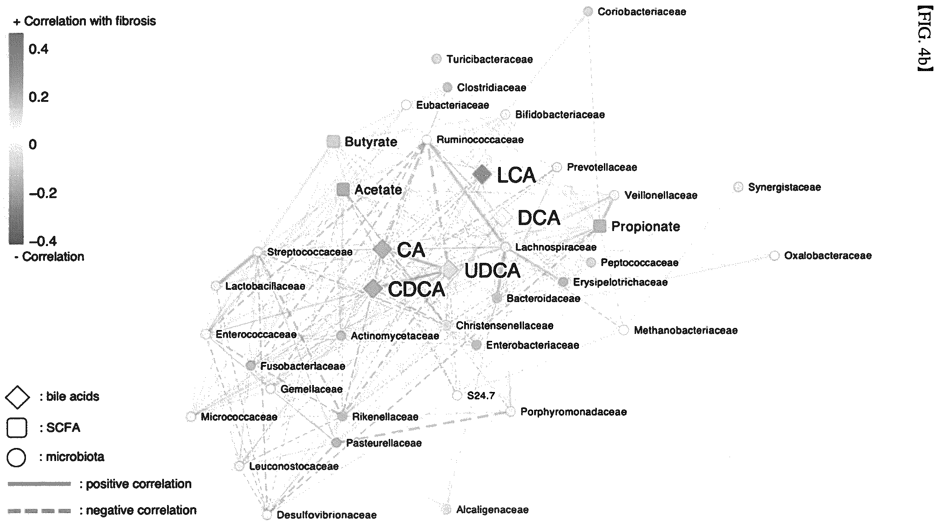

FIG. 4 is the network profile result between microbial taxa and fecal metabolite components in the non-obese (a) and obese (b) subjects. Co-expression coefficients between family-level microbiota elements and fecal metabolites were calculated using SparCC and described using Cytoscape. Solid lines (orange) and dotted lines (grey) indicate positive and negative correlations, respectively. The shape of the node indicates the components used in the present study (oval: microbiota, diamond: fecal bile acid, and round rectangle: SCFA), and the color indicates the degree of correlation according to the severity of fibrosis.
FIG. 5a and FIG. 5b are receiver operating characteristic curves (ROC) for prediction of significant fibrosis in total, non-obese and obese subjects. FIG. 5a is the ROC curve using the combination of three selected bacteria (Veillonellaceae, Ruminococcacea, and Enterobacteriaceae) and four fecal metabolites (CD, CDCA, UDCA, and propionate) drawn for prediction of significant fibrosis in all the non-obese subjects and obese subjects, and the areas under the curve (AUC) was calculated. FIG. 5b is the ROC curve using the combination of two selected bacteria (Megamonas and Ruminococcus) and four fecal metabolites (CD, CDCA, UDCA, and propionate) drawn for prediction of significant fibrosis in all the non-obese subjects and obese subjects, and the areas under the curve (AUC) was calculated.

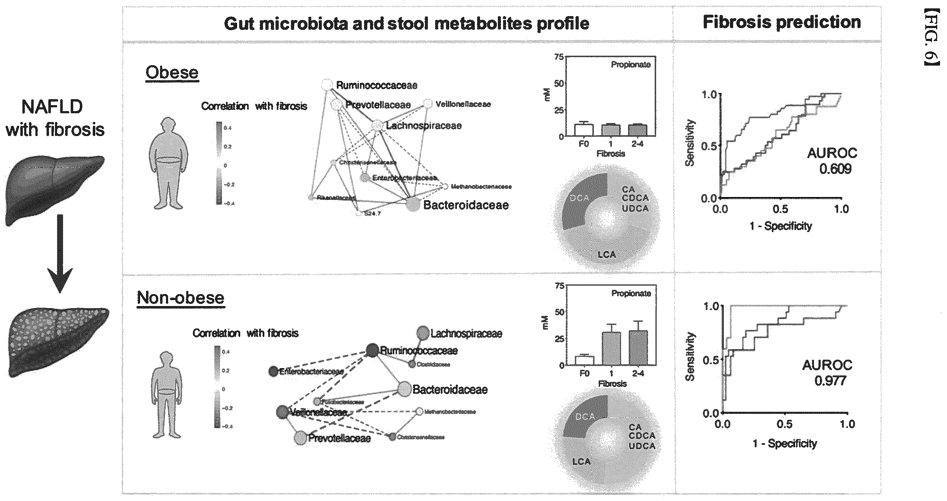

FIG. 6 is a schematic diagram that comprehensively summarizes the contents corresponding to the differences in the gut microbiome, changes in microorganisms and metabolites, and the prediction result of fibrosis through the combination of representative microorganisms and metabolites, shown in the non-obese subjects and obese subjects.

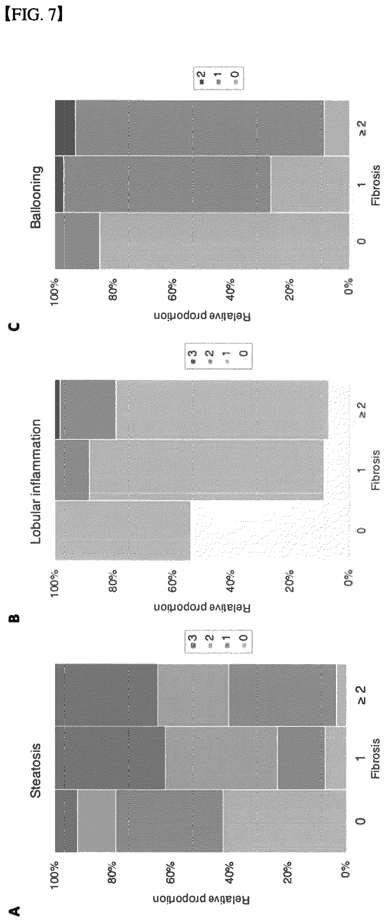

FIG. 7 is a diagram showing the histological distribution of study subjects stratified by fibrosis severity.

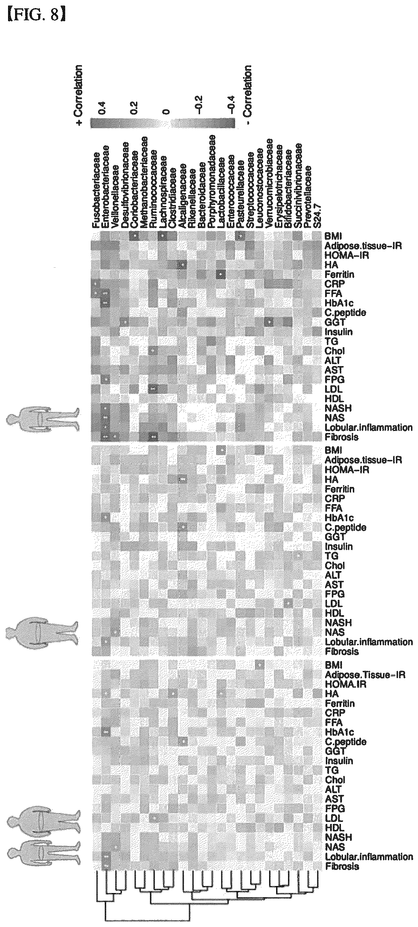

FIG. 8 is a diagram showing the correlation between the microbial taxa and metabolic indexes in the non-obese and obese patients.

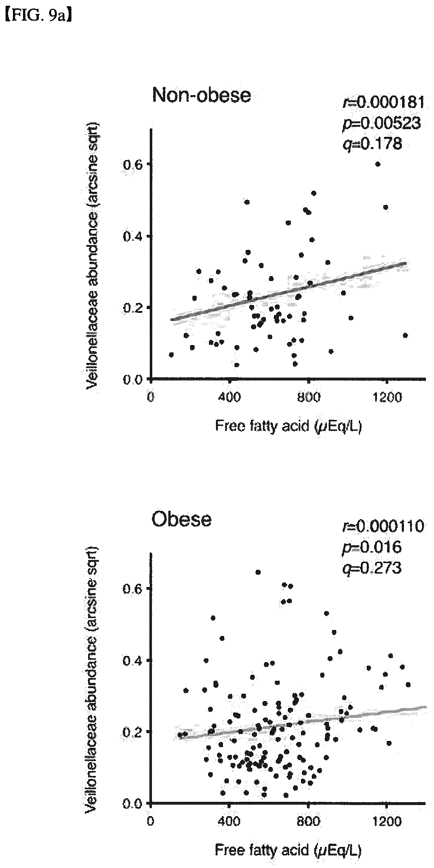

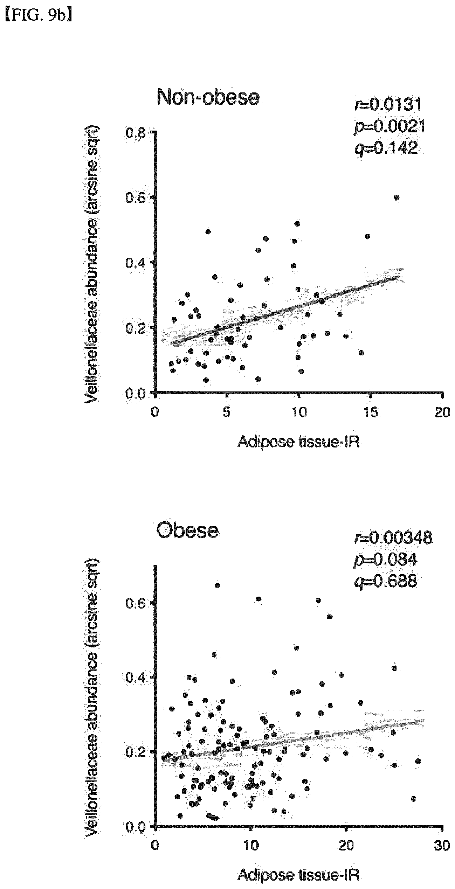

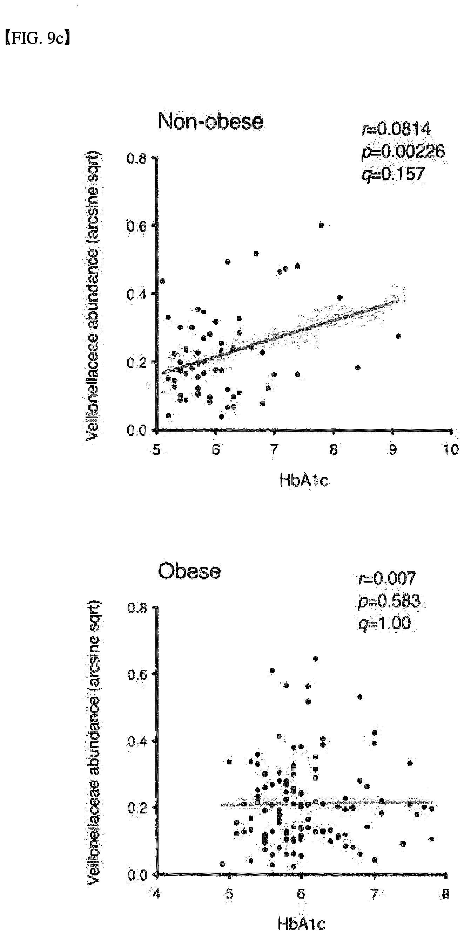

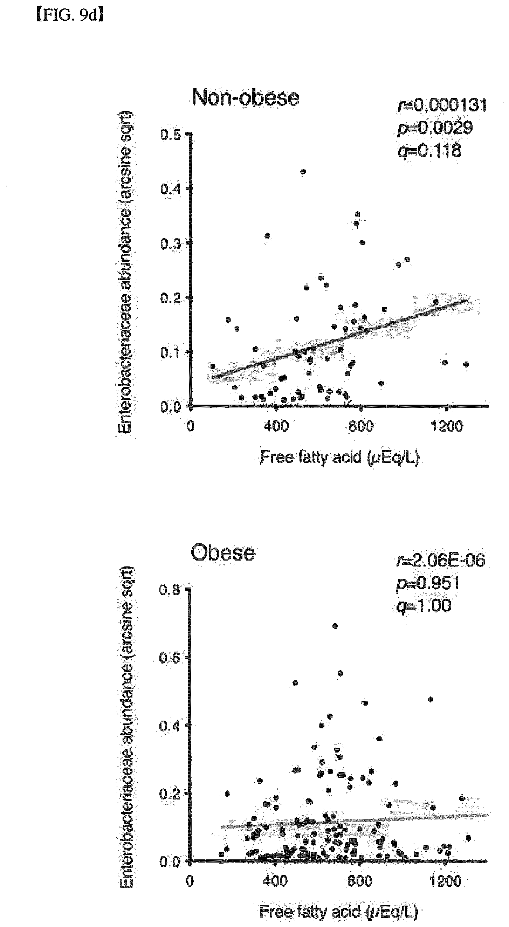

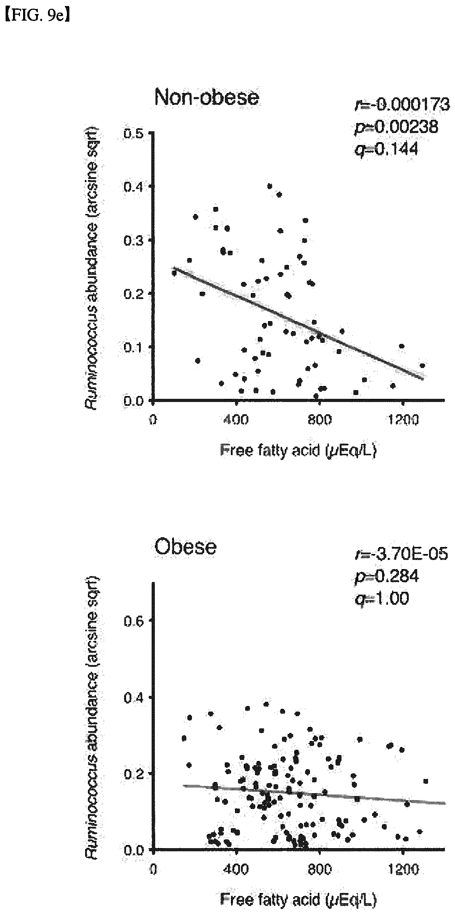

FIG. 9a to 9e are diagrams showing the correlation between the microbial taxa and metabolic indexes in the non-obese and obese patients.

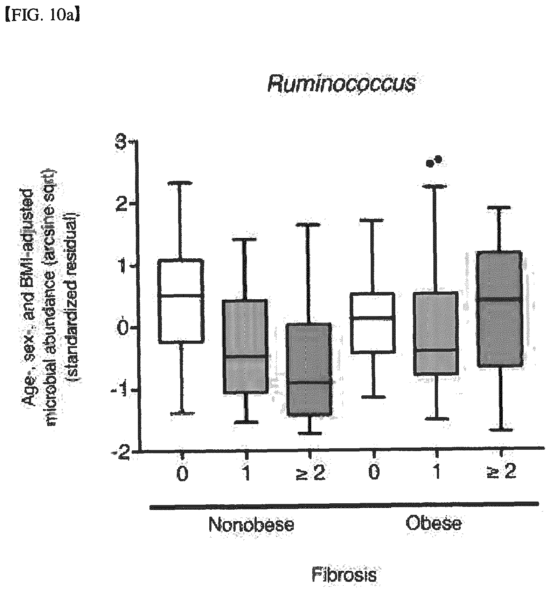

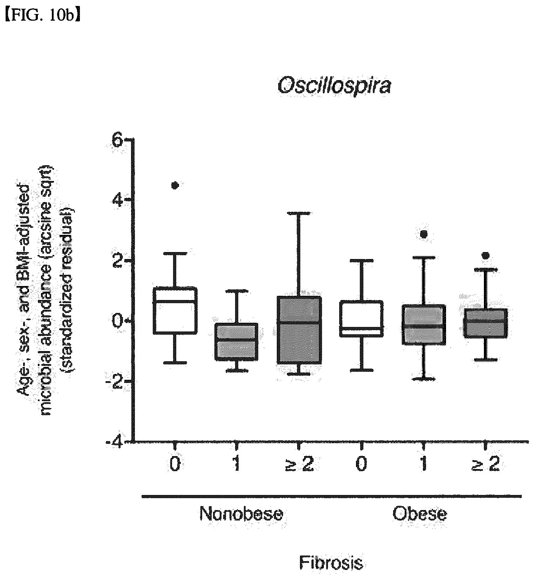

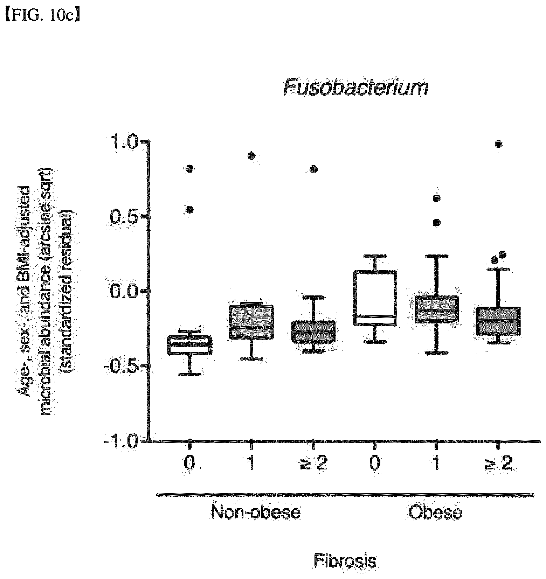

FIG. 10a to 10c are diagrams showing the relationship between the relative abundance of specific gut microbial taxa and the severity of fibrosis at the genus level stratified by the degree of obesity.

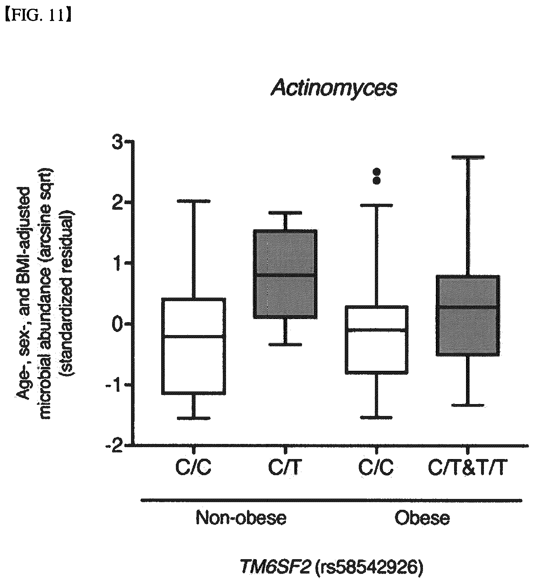

FIG. 11 is a diagram showing the relationship between the relative abundance of Actiomyces stratified by the degree of obesity and the TM6SF2 (rs58542926) variant.

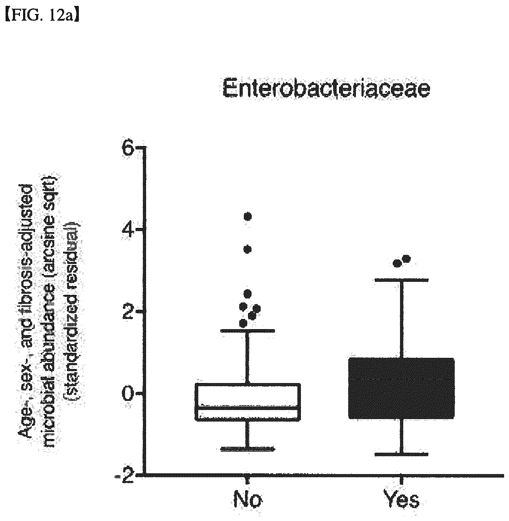

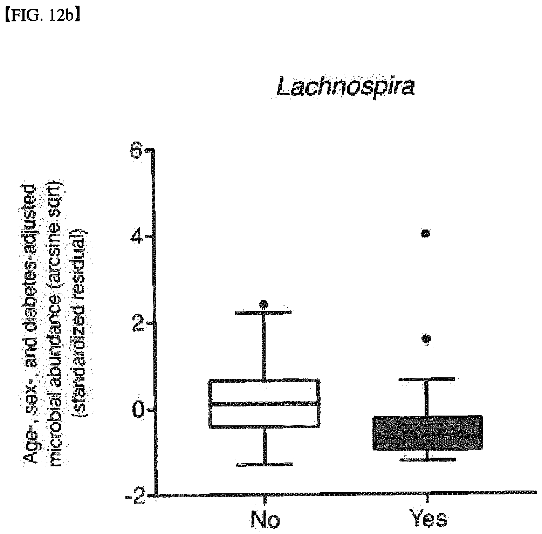

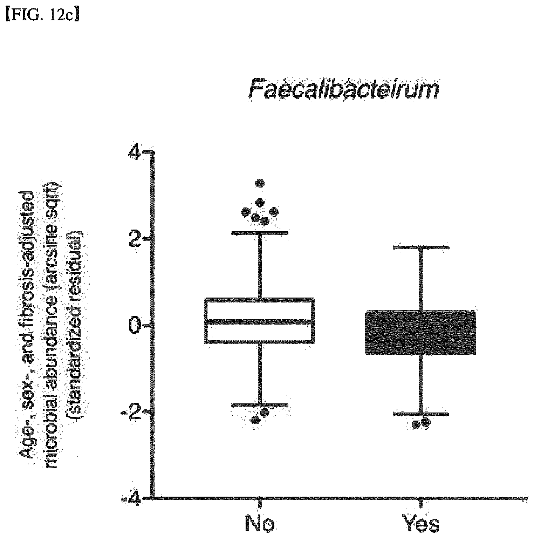

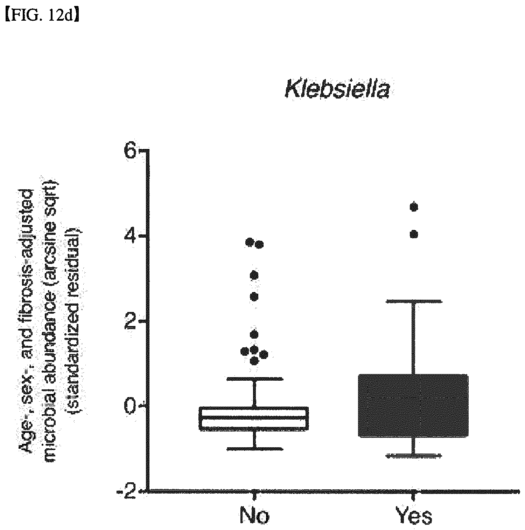

FIG. 12a to 12d are diagrams showing the relationship between the relative abundance of specific gut microbial components and the presence or absence of diabetes mellitus.

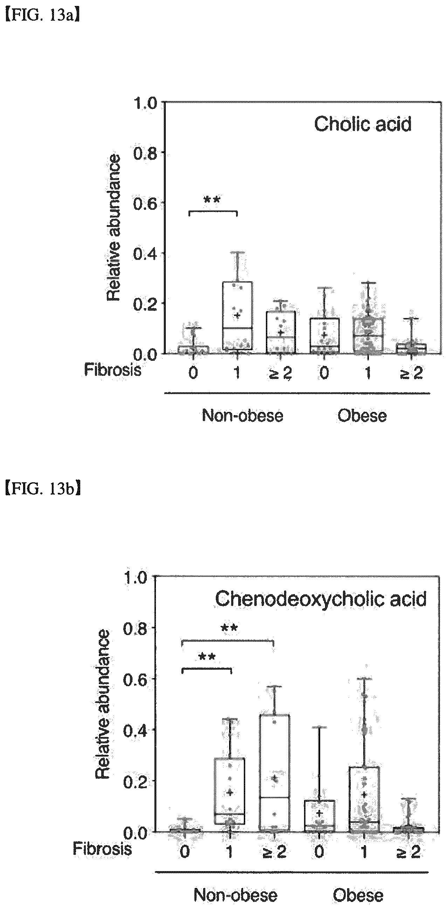

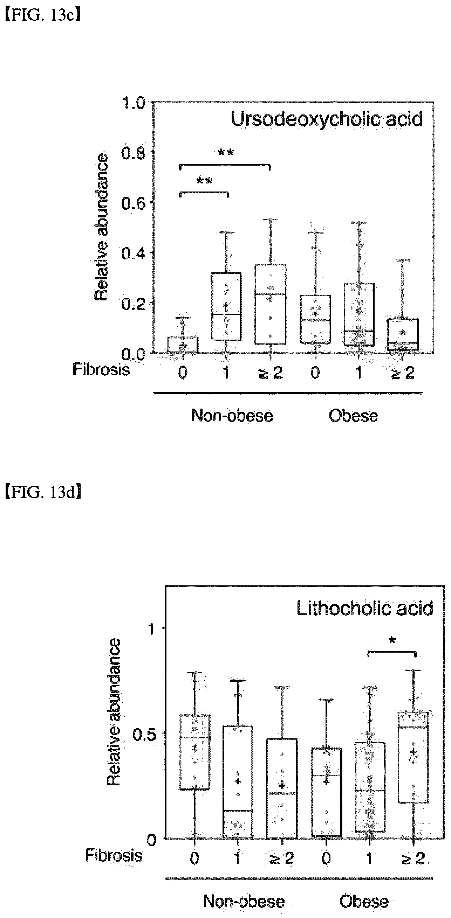

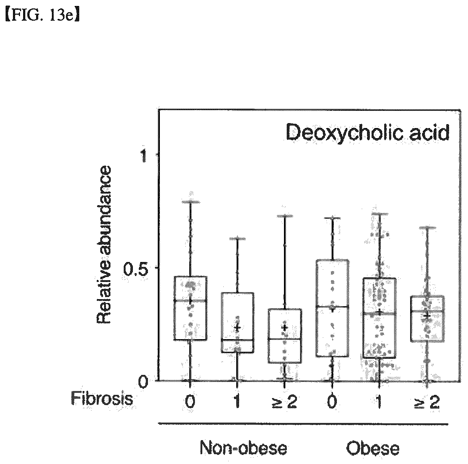

FIG. 13a to 13e are diagrams showing the relative abundance of fecal bile acid stratified by the severity of fibrosis and the degree of obesity.

### [MODE FOR INVENTION]

Hereinafter, specific Examples are provided to help the understanding of the present invention, but the following Examples are only illustrative of the present invention, and it is apparent to those skilled in the art that various changes and modifications are possible within the scope and spirit of the present invention, and it is also obvious that these changes and modifications fall within the scope of the appended claims. In the following Examples and comparative Examples, "%" and "part" indicating the content are by weight unless otherwise specified.

The values presented in the following experimental Example are expressed as means ± standard deviation (S.D.), and the statistical significance of the difference between each treatment group was determined by one-way ANOVA using Graph Pad Prism 4.0 (San Diego. CA).

### [Experimental example 1]

### 1. Material and method

### 1) Experimental subject

171 subjects demonstrated by biopsy to have NAFLD and 31 subjects without NAFLD were included. When NAFLD was confirmed histologically and BMI was BMI<25 kg/m², it was classified as the non-obese NAFLD group.

### 2) Subject inclusion and exclusion criteria

Subjects were enrolled long-term from January 2013 to February 2017, and the inclusion criteria were as follows:
1. An adult at least 18 years of age,
2. Ultrasonic findings confirming fatty infiltration of liver, and
3. An increase of alanine aminotransferase (ALT) level of unknown etiology within the past 6 months.

On the other hand, subjects who met any of the following criteria were excluded:
1. Hepatitis B or C infection,
2. Autoimmune hepatitis, primary biliary cholangitis, or primary sclerosing cholangitis,
3. Gastrointestinal cancers or hepatocellular carcinoma,
4. Drug-induced steatosis or liver damage,
5. Wilson disease or hemochromatosis,
6. Excessive alcohol consumption (male: >210g/week, female: >140g/week),
7. Antibiotic use within the previous month,
8. Diagnosis of malignancy in the past year,
9. Human immunodeficiency virus infection, and
10. Chronic disorders related to lipodystrophy or immunosuppression.

Non-obese and obese control groups included subjects without any suspicion of NFALD (a) during evaluation of living donor liver transplantation or (b) during liver biopsy for characterization of solid liver mass suspected for hepatic adenoma or focal nodular hyperplasia based on imaging studies (Koo BK, Joo SK, Kim D, Bae JM, Park JH, Kim JH, et al. Additive effects of PNPLA3 and TM6SF2 on the histological severity of non-alcoholic fatty liver disease. J Gastroenterol Hepatol 2018;33: 1277-1285.).

### 3) Liver histology

Liver histology was evaluated by a single liver pathologist using the NASH CRN histological scoring system. NAFLD was defined as the presence of ≥5% macrovesicular steatosis based on histological examination. NASH was defined based on the overall pattern of liver damage consisting of steatosis, lobular inflammation or ballooning of hepatocytes according to the criteria of Brunt et al. (Brunt EM, Janney CG, Di Bisceglie AM, Neuschwander-Tetri BA, Bacon BR. Nonalcoholic steatohepatitis: a proposal for grading and staging the histological lesions. Am J Gastroenterol 1999;94:2467-2474; Brunt EM, Kleiner DE, Wilson LA, Belt P, Neuschwander-Tetri BA. Nonalcoholic fatty liver disease (NAFLD) activity score and the histopathologic diagnosis in NAFLD: distinct clinicopathologic meanings. Hepatology 2011;53:810-820). In addition, steatosis, hepatic lobular inflammation and swelling were scored according to the NAFLD activity scoring system, and the severity of fibrosis was evaluated according to the criteria of Kleiner et al. (Kleiner DE, Brunt EM, Van Natta M, Behling C, Contos MJ, Cummings OW, et al. Design and validation of a histological scoring system for nonalcoholic fatty liver disease. Hepatology 2005;41:1313-1321).

### 4) Microbiome analysis using 16S rRNA sequencing

DNA of the fecal sample was extracted using QIAamp DNA Stool Mini Kit (Qiagen, Hilden, Germany). V4 region sequencing targeting of 16S rRNA was performed using MiSeq platform (Illumina, San Diego, CA, USA), and additional treatment of raw sequencing data was performed using QIIME pipeline (v 1.8.0) (Caporaso JG, Kuczynski J, Stombaugh J, Bittinger K, Bushman FD, Costello EK, et al. QIIME allows analysis of high-throughput community sequencing data. Nat Methods 2010;7:335-336).

### 5) Measurement of fecal metabolites using GC-FID and Q-TOP system

Fecal SCFA was measured using Agilent Technologies 7890A GC system (Agilent Technologies, Santa Clara, CA, USA) according to the method of David (David LA, Maurice CF, Carmody RN, Gootenberg DB, Button JE, Wolfe BE, et al. Diet rapidly and reproducibly alters the human gut microbiome. Nature 2014;505:559-563), and the bile acid profile was evaluated using Q-TOF mass spectrometer (Waters Micromass Technologies, Manchester, UK).

### 6) Bioinformatics analysis and statistical test

Statistical comparison was performed with Kruskal-Wallis test using GraphPad Prism software Ver. 7.0d (GraphPad Software, San Diego, CA, USA). For rarefaction curves, the OUT table was selected by 12,000 sequences per sample, and Shannon index was measured by QIIME. Nonparametric multi-dimensional scaling (NMDS) plots were represented using Vergan package of R (Oksanen J, Kindt R, Legendre P, O'Hara B, Stevens MHH, Oksanen MJ, et al. The vegan package. Community ecology package 2007;10.), and the distance was measured using Bray-Curtis method. The statistical significance between groups was estimated using Adonis function. Multivariate association analysis using microbiome data was performed using multivariate association using a linear model (MaAsLin) for identification of specific taxa related to the host phenotype without being affected by other metadata (Morgan XC, Tickle TL, Sokol H, Gevers D, Devaney KL, Ward DV, et al. Dysfunction of the gut microbiome in inflammatory bowel disease and treatment. Genome Biol 2012;13:R79.). In addition, age, gender and BMI or diabetes were designated as fixed variables, and when the *p*-value adjusted by Benjamini and Hochberg's false discovery rate (FDR) was lower than 0.20, the association rate was considered as significant.

### 7) Significant prediction of fibrosis by ROC curves

In order to demonstrate the prediction ability of fibrosis of the microbiome-based biomarkers, the area under the receiver operating characteristic curve (AUROC) method was used. The three family-level bacteria, basic characteristics of subjects (age, gender and BMI) and relative abundance of FIB-4 confirmed in the present experiment were used as inputs for AUROC, and the combination of their factors was calculated using binary logistic regression in SPSS Ver. 25.0 (SPSS Inc., Armonk, NY, USA). AUROC comparison was performed by DeLong test using MedCalc software Ver. 18.2.1 (MedCalc Software BVBA, Ostend, Belgium).

### 2. Experimental result

### 1) Basic characteristics

171 subjects demonstrated as NAFLD (NAFL, n=88; NASH, n=83) by biopsy and 31 non-NAFLD subjects were included, and all subjects were divided into two groups (non-obese, BMI<25; obese, BMI≥25), and each subject was divided into three subgroups according to the histological spectrum of NAFLD or fibrosis. In Table 1 and Table 2, the result of detailed characteristics of each group including clinical, metabolic, biochemical and histological profiles was shown.

**[Table 1]**

| Baseline characteristics of study subjects stratified by obesity status and histological spectrum of NAFLD. | | | | | | | | |
|---|---|---|---|---|---|---|---|---|
| | **Non-obese (n=64)** | | | | **Obese (n=138)** | | | |
| | **No NAFLD** | **NAFL** | **NASH** | ***P*-value** | **No NAFLD** | **NAFL** | **NASH** | ***P*-value** |
| N (male/female) | 7/14 | 13/11 | 7/12 | | 4/6 | 37/27 | 24/40 | |
| Age (years) | 58.7 ± 10.7 | 58.3 ± 10.2 | 60.2 ± 8.84 | 0.8601 ^{ns} | 58 ± 12.6 | 52.7 ± 14.8 | 53.6 ± 16.7 | 0.6463 ^{ns} |
| BMI (kg/m²) | 22.8 ± 1.67 | 23.6 ± 1.34 | 23.6 ± 0.83 | 0.0871 ^{ns} | 27 ± 2.09 | 28.8 ± 3.25 | 28.8 ± 3.02 | 0.1374 ^{ns} |
| WC (cm) | 80 ± 6.53^{a} | 82.7 ± 3.45^{ab} | 85.4 ± 4b | 0.0141 ^{∗} | 92.7 ± 5.93 | 94.3 ± 8.04 | 96.6 ± 7.81 | 0.2328 ^{ns} |
| AST (IU/L) | 31.6 ± 24.4^{a} | 28.4 ± 9.05^{b} | 54.7 ± 45.7^{bc} | 0.002 ^{∗∗} | 25.6 ± 7.5a | 42.3 ± 26.5^{b} | 62 ± 32.3^{bc} | < 0.0001 ^{∗∗∗} |
| ALT (IU/L) | 32.5 ± 32.6^{a} | 32.8 ± 17.3^{b} | 59 ± 52.9c | < 0.0001 ^{∗∗∗} | 27.8 ± 25.8^{a} | 56.9 ± 48.5^{b} | 79.1 ± 57.6^{c} | < 0.0001 ^{∗∗∗} |
| GGT (IU/L) | 44.6 ± 54^{a} | 31.8 ± 34.1^{a} | 66.7 ± 55.2^{b} | 0.0016 ^{∗∗} | 44.7 ± 51.8^{a} | 49.2 ± 57.4^{a} | 78.5 ± 79.2^{b} | < 0.0001 ^{∗∗∗} |
| HDL cholesterol (mg/dL) | 54 ± 14 | 46.3 ± 10.8 | 43.8 ± 11.7 | 0.0527 ^{ns} | 58.9 ± 14.3^{a} | 47 ± 11.6^{b} | 45.5 ± 11.2^{bc} | 0.0196 ^{∗} |
| LDL cholesterol (mg/dL) | 93.5 ± 25.7 | 111 ± 39.8 | 97.3 ± 32.1 | 0.5322 ^{ns} | 123 ± 35.8 | 103 ± 32.3 | 107 ± 32.7 | 0.1782 ^{ns} |
| Albumin (g/dL) | 4.1 ± 0.285 | 4.24 ± 0.257 | 4.03 ± 0.413 | 0.1128^{ns} | 4.12 ± 0.312 | 4.2 ± 0.254 | 4.15 ± 0.279 | 0.516 ^{ns} |
| Platelet (×10³/µL) | 223 ± 72.2^{ab} | 259 ± 54.4^{a} | 179 ± 79^{bc} | 0.0023 ^{∗∗} | 232 ± 53 | 234 ± 59.9 | 215 ± 69 | 0.3027 ^{ns} |
| Ferritin (ng/mL) | 103 ± 57.2 | 109 ± 80.2 | 173 ± 114 | 0.1285 ^{ns} | 63.8 ± 26.9^{a} | 137 ± 88.4^{b} | 159 ± 95.1^{bc} | 0.0026 |
| HA (ng/mL) | 66.6 ± 70.2^{ab} | 37.1 ± 30.2^{a} | 93.9 ± 64.9^{bc} | 0.0048 ^{∗∗} | 76.8 ± 99.2^{ab} | 62.1 ± 95.4^{a} | 95.7 ± 112^{bc} | 0.0344 ^{∗} |
| Insulin (µIU/mL) | 9.45 ± 3.77^{a} | 11.2 ± 5.96^{ab} | 13.6 ± 5.85^{b} | 0.037 ^{∗} | 11.2 ± 5.55a | 18.1 ± 17.1 ^{ab} | 18.2 ± 11.1^{b} | 0.0227 ^{∗} |
| HbAlc (%) | 5.71 ± 0.481^{a} | 6.06 ± 0.676^{a} | 7.12 ± 1.96^{b} | < 0.0001 ^{∗∗∗} | 5.72 ± 0.326^{a} | 6.1 ± 0.814^{ab} | 6.6 ± 1.27^{b} | 0.0099 ^{∗∗} |
| C-peptide (ng/mL) | 1.91 ± 0.61^{a} | 2.43 ± 0.832^{ab} | 2.88 ± 1.09^{b} | 0.0005 ^{∗∗∗} | 2.42 ± 0.916^{a} | 4.42 ± 3.4^{b} | 4.19 ± 2.39^{bc} | 0.0087 ^{∗∗} |
| HOMA-IR | 2.56 ± 1.17^{a} | 3.12 ± 1.72^{ab} | 4.39 ± 2.19^{bc} | 0.0085 ^{∗∗} | 2.92 ± 1.77^{a} | 4.96 ± 4.36^{ab} | 5.77 ± 4.42^{bc} | 0.0131 ^{∗} |
| Adipo-IR | 4.68 ± 2.85^{a} | 3.59^{ab} ± | 9.63 ± 5.39^{bc} | 0.0027 ^{∗∗} | 6.42 ± 3.28^{a} | 10.1 ± 10.1^{ab} | 12.8 ± 9.51^{bc} | 0.0096 ^{∗∗} |
| FFA (µEq/L) | 493 ± 166^{ab} | 620 ± 240^{a} | 720 ± 268^{bc} | 0.0121 ^{∗} | 605 ± 289a | 603 ± 259^{ab} | 712 ± 238^{bc} | 0.0089 ^{∗∗} |
| hsCRP (mg/dL) | 0.249 ± 0.428^{a} | 0.0896 ± 0.066^{a} | 0.354 ± 0.549^{b} | ± 0.0119 ^{∗} | 0.152 ± 0.154^{ab} | 0.206 ± 0.403^{a} | 0.278 ± 0.336^{bc} | 0.0294 ^{∗} |
| Cholesterol (m/dL) | 167 ± 28.2 | 185 ± 41.8 | 167 ± 42.9 | 0.3932 ^{ns} | 200 ± 43.2 | 183 ± 34.8 | 181 ± 40.7 | 0.4122 ^{ns} |
| TG (mg/dL) | 102 ± 47^{a} | 140 ± 44.7^{b} | 141 ± 70.9^{ab} | 0.0105 ^{∗} | 87 ± 34^{a} | 161 ± 82.2^{b} | 151 ± 61.9^{bc} | 0.0024 ^{∗∗} |
| FPG (mg/dL) | 110 ± 25.6 | 113 ± 28.8 | 132 ± 42.5 | 0.0986 ^{ns} | 102 ± 14.1 | 113 ± 27.4 | 128 ± 55.8 | 0.2074 ^{ns} |
| HTN, n (%) | 8 (38.1) | 8 (33.3) | 9 (47.4) | 0.641 ^{ns} | 4 (40.0) | 24 (37.5) | 32 (50.0) | 0.352 ^{ns} |
| Diabetes, n (%) | 1 (4.76) | 8 (33.3) | 13 (68.4) | 0.0001 ^{∗∗∗} | 1 (10.0) | 19 (29.7) | 30 (46.9) | 0.026 ^{∗} |

| | | | | | | | | |
|---|---|---|---|---|---|---|---|---|
| Abbreviations: BMI, body mass index; WC, waist circumference; AST, aspartate transaminase; ALT, alanine transaminase; GGT, gamma-glutamyl transferase; NAS, nonalcoholic fatty liver disease activity score; HDL, high-density lipoprotein; LDL, low-density lipoprotein; HA, hyaluronic acid; HbAlc, glycosylated hemoglobin; HOMA-IR, homeostasis model assessment of insulin resistance; Adipo-IR, adipose tissue insulin resistance; FFA, free fatty acid; hsCRP, high-sensity C-reactive protein; TG, triglycerides; FBG, fasting blood glucose; HTN, hypertension. Data are expressed as the mean ± SD or n (%). Mean ± SD or n (%) with defferent superscript letters indicates significant differences by the nonparametric Kruskal-Wallis test or the chi-square test. ^{∗}P<0.05, ^{∗∗}P<0.01, ^{∗∗∗}P<0.001 | | | | | | | | |

**[Table 2]**

| Baseline characteristics of study subjects stratified by obesity status and fibrosis severity. | | | | | | | | |
|---|---|---|---|---|---|---|---|---|
| | **Non-obese (n=64)** | | | | **Obese (n=138)** | | | |
| **Fibrosis stage** | **0** | **1** | **≥ 2** | ***P*-value** | **0** | **1** | **2** | ***P*-value** |
| N (male/female) | 27 (11/16) | 20 (9/11) | 17 (7/10) | | 25 (17/8) | 73 (38/35) | 40 (10/30) | |
| Age (years) | 57.67 ± 9.01 | 57.85 ± 11.80 | 62.47 ± 8.32 | 0.2371 ^{ns} | 5 7.08 ± 12.41^{ab} | 48.36 ± 15.70^{a} | 60.63 ± 13.56^{b} | 0.0001 ^{∗∗∗} |
| BMI (kg/m²) | 22.81 ± 1.42^{a} | 23.16 ± 1.46^{b} | 23.71 ± 0.92^{ab} | 0.0084 ^{∗∗} | 27.48 ± 2.58^{a} | 29.30 ± 3.20^{b} | 28.27 ± 2.97^{ab} | 0.0119 ^{∗} |
| WC (cm) | 79.95 ± 5.2^{a} | 83.22 ± 3.53^{ab} | 86.33 ± 4.22^{b} | 0.0010 ^{∗∗} | 91.82 ± 6.56 | 96.16 ± 7.35 | 95.73 ± 8.98 | 0.0074 ^{ns} |
| AST (IU/L) | 29.11 ± 21.64^{a} | 32.20 ± 10.63^{ab} | 56.06 ± 48.13^{b} | 0.0017 ^{∗∗} | 28.40 ± 13.03^{a} | 48.99 ± 26.74^{b} | 66.13 ± 36.43^{c} | < 0.0001 ^{∗∗∗} |
| ALT (IU/L) | 31.89 ± 27.83 | 39.10 ± 30.99 | 55.71 ± 52.04 | 0.0886 ^{ns} | 38.88 ± 43.67^{a} | 70.85 ± 53.02^{bc} | 70.85 ± 56.63^{c} | 0.0003 ^{∗∗∗} |
| GGT (IU/L) | 33.6 ± 41.4^{a} | 44 ± 46.2^{ab} | 69.7 ± 58.3^{b} | 0.0046 ^{∗∗} | 39.6 ± 41^{a} | 57.5 ± 56.1^{ab} | 85.9 ± 95.2^{b} | 0.0016 ^{∗∗} |
| HDL-cholesterol (mp/dL) | 51.4 ± 13.4 | 47.9 ± 11.1 | 43.1 ± 12.3 | 0.1701 ^{ns} | 48.4 ± 13.1 | 47.2 ± 12 | 46.3 ± 11.5 | 0.9175 ^{ns} |
| LDL-cholesterol (mg/dL) | 104 ± 29.9 | 108 ± 38.6 | 90.8 ± 32.3 | 0.3250 ^{ns} | 105 ± 35.7 | 109 ± 31.7 | 102 ± 33.8 | 0.5024 ^{ns} |
| Albumin (g/dL) | 4.14 ± 0.25 | 4.22 ± 0.29 | 3.99 ± 0.43 | 0.1781 ^{ns} | 4.12 ± 0.24^{ab} | 4.24 ± 0.26^{a} | 4.08 ± 0.27^{b} | 0.0027 ^{∗∗} |
| Platelet (×10³/µL) | 230.19 ± 48.76^{a} | 247.75 ± 74.84^{a} | 183.88 ± 95.35^{b} | 0.0255 ^{∗} | 238.2 ± 55.38^{a} | 241.44 ± 62.49^{a} | 188.33 ± 58.05^{b} | 0.0001 ^{∗∗∗} |
| Ferritin (ng/mL) | 117.75 ± 73.97 | 100.94 ± 74.93 | 282.19 ± 386.91 | 0.053 ^{ns} | 145.55 ± 89.51 | 219.37 ± 255.97 | 169.26 ± 133.32 | 0.8403 ^{ns} |
| HA (ng/mL) | 33.08 ± 19.62^{a} | 64.2 ± 67.47^{a} | 109.59 ± 65.56^{b} | 0.0002 ^{∗∗∗} | 51.32 ± 66.4^{a} | 61.58 ± 90.48^{a} | 127.28 ± 129.4^{b} | 0.0001 ^{∗∗∗} |
| Insulin (µIU/mL) | 10.76 ± 5.57 | 10.05 ± 4.15 | 13.71 ± 6.19 | 0.1103 ^{ns} | 14.22 ± 11.57^{a} | 17.83 ± 15.46^{ab} | 19.50 ± 12.58^{b} | 0.0148 ^{∗} |
| HbAlc (%) | 5.86 ± 0.69^{a} | 5.98 ± 0.44^{ab} | 7.23 ± 2.05^{c} | 0.0007 ^{∗∗∗} | 5.87 ± 0.54^{a} | 6.15 ± 0.85^{ab} | 6.87 ± 1.42^{c} | 0.0007 ^{∗∗∗} |
| C-peptide (ng/mL) | 2.23 ± 0.87^{a} | 2.23 ± 0.64^{ab} | 2.85 ± 1.17^{bc} | 0.0256 ^{∗} | 3.22 ± 1.57^{a} | 4.43 ± 3.43^{ab} | 4.29 ± 2.28^{bc} | 0.0498 ^{∗} |
| HOMA-IR | 2.94 ± 1.61^{a} | 2.81 ± 1.33^{ab} | 4.50 ± 2.28^{b} | 0.0207 ^{∗} | 3.84 ± 3.35^{a} | 4.82 ± 4.18^{ac} | 6.69 ± 4.70^{b} | 0.0006 ^{∗∗∗} |
| Adipo-IR | 5.72 ± 3.01 | 6.33 ± 3.72 | 9.49 ± 5.95 | 0.0645 ^{ns} | 7.48 ± 6.2^{a} | 10.76 ± 9.7^{ab} | 13.86 ± 10.55^{bc} | 0.0043 ^{∗∗} |
| FFA (µEq/L) | 553.96 ± 186.66 | 615.65 ± 238.13 | 684.88 ± 308.55 | 0.2678 | 556.08 ± 209.52^{a} | 642.76 ± 257.61^{ab} | 737.1 ± 259.42^{bc} | 0.0059 ^{∗∗} |
| hsCRP (mg/dL) | 0.17 ± 0.33^{a} | 0.23 ± 0.41^{ab} | 0.29 ± 0.48^{bc} | 0.0186 ^{∗} | 0.14 ± 0.17^{a} | 0.23 ± 0.39^{b} | 0.3 ± 0.39^{bc} | 0.0121 ^{∗} |
| Cholesterol (mg/dL) | 177.7 ± 28 | 180.75 ± 43.68 | 158.53 ± 44.94 | 0.1860 ^{ns} | 180.96 ± 38.04 | 188.58 ± 34.01 | 175.33 ± 4.67 | 0.2143 ^{ns} |
| TG (mg/dL) | 120.70 ± 45.23 | 128.42 ± 51.84 | 137.12 ± 77.04 | 0.9889 ^{ns} | 127.36 ± 51.42 | 156.23 ± 80.68 | 155.43 ± 67.19 | 0.2363 ^{ns} |
| FPG (mg/dL) | 111.15 ± 31.51^{a} | 111.85 ± 19.58^{ab} | 134.47 ± 43.79^{b} | 0.0402 ^{∗} | 110.96 ± 34.36^{a} | 107.82 ± 21.43^{a} | 144.53 ± 63.53^{b} | 0.0001 ^{∗∗∗} |
| HTN, n (%) | 7 (25.9) | 9 (45.0) | 9 (52.9) | 0.163 ^{ns} | 9 (36.0) | 30 (41.1) | 21 (52.5) | 0.357 ^{ns} |
| Diabetes, n (%) | 4 (14.8) | 5 (25.0) | 13 (76.5) | 0.0001 ^{∗∗∗} | 3 (12.0) | 23 (31.5) | 24 (60.0) | 0.0002 ^{∗∗∗} |

As a result of confirmation, subjects with NASH or significant fibrosis (F2-4) had high levels of aspartate aminotransferase (AST), alanine aminotransferase (ALT) and diabetic markers in all obese and non-obese groups. The subjects with significant fibrosis had higher NAFLD activity scores, and showed more severe liver histology in terms of histological classification of NAFLD (Table 3 and FIG. 7). More detailed standard characteristics of each fibrosis stage, comprising well-known NAFLD-related genetic variations such as PNPLA3, TM6SF2, MBOAT7-TMC4, and SREBF-2 were shown in Table 4.

**[Table 3]**

| Histological characteristics of study subjects stratified by obesity status and fibrosis severity. | | | | | | |
|---|---|---|---|---|---|---|
| | **Non-obese (n=64)** | | | **Obese (n=138)** | | |
| **Fibrosis stage** | **0** | **1** | **2** | **0** | **1** | **2** |
| Steatosis, n (%) | | | | | | |
| 0 (<5%) | 15 (55.6) | 5 (25.0) | 1 (5.9) | 7 (28.0) | 2 (2.7) | 1 (2.5) |
| 1 (5-33%) | 7 (25.9) | 6 (30.0) | 8 (47.1) | 12 (48.0) | 9 (12.3) | 13 (32.5) |
| 2 (34-66%) | 4 (14.8) | 7 (35.0) | 2(11.8) | 3 (12.0) | 29 (39.7) | 12 (30.0) |
| 3 (>66%) | 1 (3.7) | 2 (10.0) | 6 (35.3) | 3 (12.0) | 33 (45.2) | 14 (35.0) |
| Lobular inflammation, n (%) | | | | | | |
| 0 | 15 (55.6) | 3 (15.0) | 1 (5.9) | 13 (52.0) | 5 (6.9) | 3 (7.5) |
| 1 | 12 (44.4) | 14 (70.0) | 11 (64.7) | 12 (48.0) | 60 (82.2) | 30 (75.0) |
| 2-3 | 0 | 3 (15.0) | 5 (29.4) | 0 | 8 (11.0) | 7 (17.5) |
| Ballooning, n (%) | | | | | | |
| 0 | 22 (81.5) | 7 (35.0) | 0 | 22 (88.0) | 18 (24.7) | 5 (12.5) |
| 1-2 | 5 (18.5) | 13 (65.0) | 17(100.0) | 3 (12.0) | 55 (75.3) | 35 (87.5) |
| Histological classification, n (%) | | | | | | |
| No NAFLD | 15 (55.6) | 5 (25.0) | 1 (5.9) | 7 (28.0) | 2 (2.7) | 0 |
| NAFL | 11 (40.7) | 13 (65.0) | 0 | 18(72.0) | 40 (54.8) | 7(17.5) |
| NASH | 1 (3.7) | 2 (10.0) | 16(94.1) | 0 | 31 (42.5) | 33 (82.5) |
| NAS | 1.30 ± 1.46 | 2.95 ± 1.61 | 4.00 ± 1.32 | 1.68 ± 1.22 | 4.11 ± 1.23 | 4.08 ± 1.10 |

| | | | | | | |
|---|---|---|---|---|---|---|
| Abbreviations: NAFLD, nonalcoholic fatty liver disease; NAFL, nonalcoholic fatty liver; NASH, nonalcoholic steatohepatitis; NAS, NAFLD activity score. Date are expressed as the mean ± SD or n (%). | | | | | | |

**[Table 4]**

| Baseline clinical, metabolic, histological, and genetic characteristics of study subjects stratified by obesity status and fibrosis stage. | | | | | | | | | | | |
|---|---|---|---|---|---|---|---|---|---|---|---|
| | | **Non-obese (n=64)** | | | | | **Obese (n=138)** | | | | |
| **Fibrosis stage** | | **0** | **1** | **2** | **3** | **4** | **0** | **1** | **2** | **3** | **4** |
| N (male/female) | | 27 (11/16) | 20 (9/11) | 9(5/4) | 4(1/3) | 4(1/3) | 25 (17/8) | 73 (38/35) | 20 (5/15) | 7 (2/5) | 13 (3/10) |
| Age (years) | | 57.7 ± 9.01 | 57.8 ± 11.8 | 58.6 ± 9.9 | 67.2 ± 2.5 | 66.5 ± 1.73 | 57.1 ± 12.4 | 48.4 ± 15.7 | 55.6 ± 16.4 | 65.6 ± 8.89 | 65.8 6.92 |
| BMI | | 22.8 ± 1.42 | 23.8 ± 1.46 | 23.5 ± 0.862 | 23.9 ± 1.33 | 24 ± 0.66 | 27.5 ± 2.58 | 29.3 ± 3.2 | 28.5 ± 3.26 | 28 ± 3.43 | 28 ± 2.39 |
| WC (cm) | | 79.9 ± 5.2 | 83.2 ± 3.53 | 85.5 ± 2.27 | 88.1 ± 4.3 | 86.1 ± 8.37 | 91.8 ± 6.55 | 96.2 ± 7.35 | 95.8 ± 9.02 | 95.8 ± 13.2 | 95.6 ± 7.76 |
| SBP (mm Hg) | | 128 ± 16.9 | 127 ± 14.3 | 132 ± 17.1 | 158 ± 37.4 | 124 ± 21.7 | 130 ± 14.8 | 136 ± 18.4 | 133 ± 17 | 132 ± 11.3 | 126 ± 17.9 |
| DBP (mm Hg) | | 76.8 ± 12.8 | 77.4 ± 8.26 | 80.2 ± 12.9 | 86.8 ± 19.1 | 74.5 ± 10.8 | 80 ± 9.78 | 84.1 ± 11.8 | 77.4 ± 13.4 | 77 ± 10.8 | 74.3 ± 9.55 |
| AST (IU/L) | | 29.1 ± 21.6 | 32.2 ± 10.6 | 55.1 ± 55.8 | 80.5 ± 50.1 | 33.8 ± 8.34 | 28.4 ± 13 | 49 ± 26.7 | 66.6 ± 46.5 | 70.4 ± 27.9 | 63 ± 21.7 |
| ALT (IU/L) | | 31.9 ± 27.8 | 39.1 ± 31 | 52.6 ± 53.2 | 93.5 ± 60.5 | 25 ± 7.53 | 38.9 ± 43.7 | 70.8 ± 53 | 87.2 ± 74.3 | 58 ± 19.3 | 52.6 24.6 |
| GGT (IU/L) | | 33.6 ± 41.4 | 44 ± 46.2 | 69.7 ± 64 | 373 ± 592 | 63 ± 43.8 | 39.6 ± 41 | 57.5 ± 56.1 | 79.4 ± 86.1 | 86.6 ± 68.5 | 95.6 123 |
| Insulin (µIU/mL) | | 10.8 ± 5.57 | 10 ± 4.15 | 15 ± 6.14 | 15.3 ± 7.18 | 9.32 ± 4.29 | 14.2 ± 11.6 | 17.8 ± 15.5 | 21.3 ± 16.1 | 13.5 ± 4.73 | 20 ± 8.1 |
| HbAlc (%) | | 5.86 ± 0.688 | 5.98 ± 0.445 | 6.8 ± 0.689 | 6.05 ± 0.557 | 9.38 ± 3.52 | 5.87 ± 0.538 | 6.15 ± 0.851 | 7.01 ± 1.45 | 6.79 ± 1.15 | 6.74 ± 1.59 |
| HOMA-IR | | 2.94 ± 1.61 | 2.81 ± 1.33 | 4.57 ± 2.05 | 4.56 ± 2.04 | 4.27 ± 3.5 | 3.84 ± 3.36 | 4.82 ± 4.18 | 7.28 ± 5.79 | 4.4 ± 1.42 | 7.01 ± 3.71 |
| Adipo-IR | | 5.72 ± 3.01 | 6.33 ± 3.72 | 11.1 ± 6.82 | 8.94 ± 5.18 | 6.44 ± 4.2 | 7.48 ± 6.2 | 10.8 ± 9.7 | 14.3 ± 12.9 | 9.29 ± 7.57 | 15.1 ± 7.42 |
| Diabetes, n (%) | | 4 (14.8) | 5 (25.0) | 7 (77.8) | 2 (50.0) | 4 (100) | 3 (12.0) | 23 (31.5) | 12 (60.0) | 4(57.1) | 8 (61.5) |
| Albumin (g/dL) | | 4.15 ± 0.259 | 4.22 ± 0.291 | 4.19 ± 0.285 | 3.92 ± 0.45 | 3.62 ± 0.499 | 4.12 ± 0.243 | 4.24 ± 0.26 | 4.11 ± 0.192 | 4.06 ± 0.207 | 4.04 ± 0.393 |
| Platelet (×10³/µL) | | 230 ± 48.8 | 248 ± 74.8 | 222 ± 67.2 | 206 ± 123 | 76.8 ± 32.1 | 238 ± 55.4 | 241 ± 62.5 | 206 ± 50.6 | 195 ± 48.8 | 157 ± 63.7 |
| TG (mg/dL) | | 121 ± 45.2 | 128 ± 51.8 | 180 ± 82.9 | 82.5 ± 26.6 | 96 ± 31.1 | 127 ± 51.4 | 156 ± 80.7 | 175 ± 71.5 | 140 ± 48.6 | 134 ± 64.5 |
| FPG (mg/dL) | | 111 ± 31.5 | 112 ± 19.6 | 123 ± 26.7 | 123 ± 37 | 172 ± 66.6 | 111 ± 34.4 | 108 ± 21.4 | 148 ± 78.3 | 138 ± 30.6 | 143 ± 53.9 |

| **Histological classification** | | | | | | | | | | | |
|---|---|---|---|---|---|---|---|---|---|---|---|
| No NAFLD | | 15 (55.6) | 5 (25.0) | 0 | 1 (25.0) | 0 | 7 (28.0) | 2 (2.7) | 0 | 0 | 0 |
| NAFL | | 11(40.7) | 13 (65.0) | 0 | 0 | 0 | 18 (72.0) | 40 (54.8) | 6 (30.0) | 1 (14.3) | 0 |
| NASH | | 1 (3.7) | 2 (10.0) | 9(100) | 3 (75.0) | 4 (100) | 0 | 31 (42.5) | 14 (70.0) | 6 (85.7) | 13 (100) |

| **Genetic variants** | | | | | | | | | | | |
|---|---|---|---|---|---|---|---|---|---|---|---|
| *PNPLA3* (rs738409) | G/G | 6 (22.2) | 4 (20.0) | 1 (11.1) | 0 | 2 (50.0) | 4 (27.4) | 20 (27.4) | 11 (55.0) | 2 (28.6) | (46.2) |
| | C/G | 13 (38.1) | 13 (65.0) | 5 (55.6) | 1 (25.0) | 2 (50.0) | 11 (44.0) | 35(47.9) | 4 (20.0). | (57.1) | 4 (30.8) |
| | C/C | 7 (25.9) | 3 (15.0) | 2 (22.2) | 3 (75.0) | 0 | 7 (17.8) | 13 (17.8) | 4 (20.0) | 1 (14.3) | 1 (7.7) |
| *TM6SF2* (rs58542926) | C/C | 21 (77.8) | 18 (90.0) | 6 (66.7) | 2 (50.0) | 3 (75.0) | 18 (72.0) | 56 (76.7) | 16 (80.0) | 4 (57.1) | 10 (76.9) |
| | C/T | 5 (18.5) | 2 (10.0) | 2 (22.2) | 2 (50.0) | 1 (25.0) | 4 (16.0) | 11 (15.1) | 3 (15.0) | 2 (28.6) | 1 (7.7) |
| | T/T | 0 | 0 | 0 | 0 | 0 | 0 | 1 (1.4) | 0 | 1 (14.3) | 0 |
| *MBOAT7-TMC4* (rs641738) | C/C | 17 (63.0) | 13 (65.0) | 5 (55.6) | 1 (25.0) | 4 (100) | 15 (60.0) | 42 (57.5) | 11 (55.0) | 3 (42.9) | 7 (53.8) |
| | C/T | 9 (33.3) | 5 (25.0) | 3 (33.3) | 3 (75.0) | 0 | 4 (16.0) | 21 (28.8) | 8 (40.0) | 4 (57.1) | 4 (30.8) |
| | T/T | 0 | 2 (10.0) | 0 | 0 | 0 | 3 (12.0) | 5 (6.8) | 0 | 0 | 0 |
| *SREBF-2* (rs133291) | C/C | 7 (25.9) | 6 (30.0) | 1 (11.1) | 2 (50.0) | 2 (50.0) | 6 (24.0) | 23 (31.5) | 8 (40.0) | 1 (14.3) | 3 (23.1) |
| | C/T | 12 (44.4) | 8 (40.0) | 6 (66.7) | 1 (25.0) | 2 (50.0) | 7 (28.0) | 27 (37.0) | 5 (25.0) | 5 (71.4) | 5 (38.5) |
| | T/T | 3(11.1) | 6 (30.0) | 1 (11.1) | 1 (25.0) | 0 | 3 (12.0) | 8 (11.0) | 4 (20.0) | 1 (14.3) | 2 (15.4) |

| | | | | | | | | | | | |
|---|---|---|---|---|---|---|---|---|---|---|---|
| Abbreviations: BMI, body mass index; WC, waist circumference; SBP, systolic blood pressure; DBP, diastolic blood pressure; AST, aspartate transaminase; ALT, alanine transaminase; GGT, gamma-glutamyl transferase; HbA1c, glycosylated hemoglobin; HOMA-IR, homeostasis model assessment of insulin resistance; Adipo-IR, adipose tissue insulin resistance; TG, triglycerides; FBG, fasting blood glucose; NAFLD, nonalcoholic fatty liver disease; NASH, nonalcoholic steatohepatitis; PNPLA3, patatin-like phospholipase domain-containing protein 3; TM6SF2, transmembrane 6 superfamily 2; MBOAT7-TMC4, membrane bound O-acyltransferase domain-containing 7 gene and transmembrane channel-like 4 gene; SREBF-2, sterol regulatory element binding transcription factor 2. Data are expressed as mean ± SD or n (%). | | | | | | | | | | | |

### 2) Observation of changes in microbiome according to fibrosis severity

Depending on the fibrosis severity, changes of the microbiome were shown differently in the non-obese NAFLD subjects and obese NAFLD subjects.

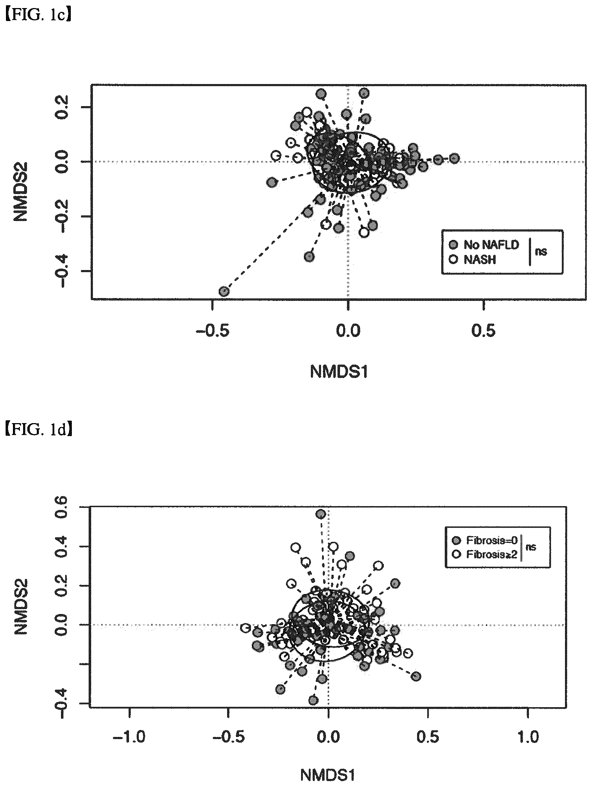

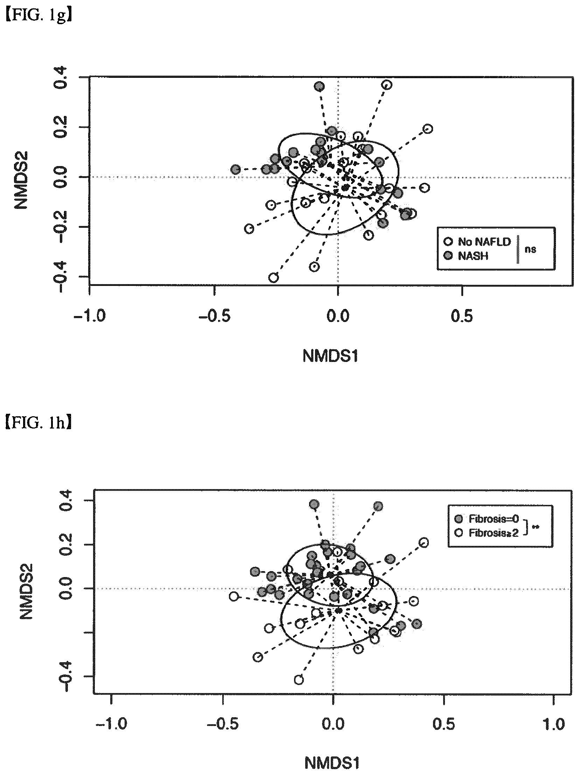

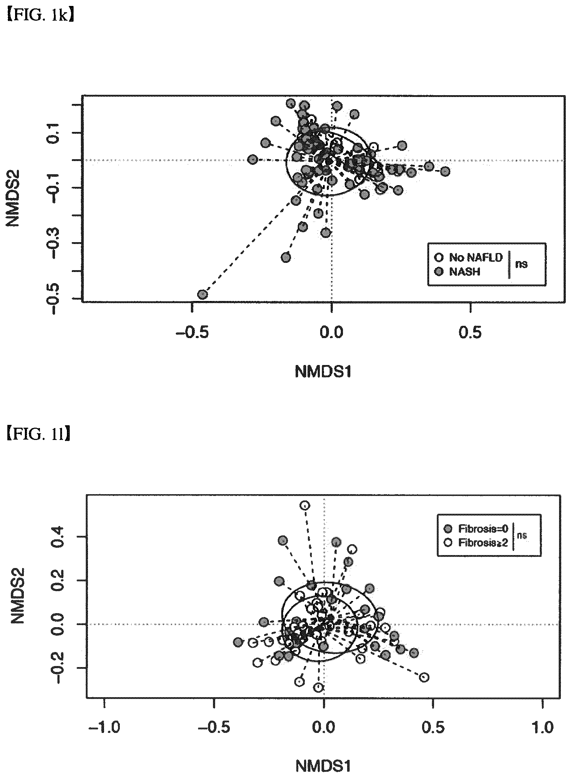

Specifically, the microbial diversity was compared according to the histological spectrum of NAFLD or fibrosis severity (FIG. 1). For comparison of alpha diversity, rarefaction curves based on Shannon metric were plotted, and NMDS plots based on Bray-Curtis distance were plotted for beta diversity. As a result of confirmation, any significant changes between groups stratified by the histological spectrum of NAFLD or fibrosis severity were not found in the merged subjects (FIG. 1a to 1d).

The subjects were classified into two groups according to their BMI status. In the non-obese group, a significant decrease in microbial diversity was observed between F1 and F0 (*p*=0.0074), as well as between F2-4 and F0 (*p*=0.0084) (FIG. 1e to 1h). Moreover, clear clustering between F0 and F2-4 was observed (p=0.038). In the obese group, there was no significant change in diversity between groups stratified by the histological classification of NAFLD or fibrosis severity (FIG. 1i to 11).

The result indicates that the fibrosis severity is more related to gut microbiome change than necroinflammatory activity, and basic BMI status may also be an important factor contributing to gut microbiome change.

### 3) Observation of proliferation of fibrosis-related microbial taxa

Proliferation of the fibrosis-related microbial taxa was remarkably shown in the non-obese NAFLD subjects. Specifically, in the non-obese and obese subjects, the differences of the specific microbial taxa according to the fibrosis severity were compared using univariate and multivariate analyses (FIG. 2a to 2d and 2e to 2h).

In the univariate analysis, not only gradual proliferation of Veillonellaceae mostly found in the oral cavity and small intestine and large intestine, but also Enterobacteriaceae were observed according to the fibrosis severity of the non-obese subjects. In the obese subjects, Rikenellaceae became gradually enriched. On the contrary, the abundance of Ruminococcaceae was significantly reduced as fibrosis became more severe, and this was found only in the non-obese subjects. This result could be confirmed in correlation plots (FIG. 8), and Enterobacteriaceae and Veillonellaceae showed a positive correlation with the fibrosis severity (*p*=1.09×10⁻⁴, *p*=2.44×10⁻³, respectively), but Ruminococcaceae showed an inverse correlation.

At the genus level, Faecalibacterium (Ruminococcaceae), Ruminococcus (Ruminococcaceae), Coprococcus (Lachnospiraceae), and Lachnospira (Lachnospiraceae) were significantly drastically reduced in the significant fibrosis group, but the abundance of Enterobacteriaceae_Other (Enterobacteriaceae) and Citrobacter was gradually increased according to the fibrosis severity. This change was observed only in the non-obese subjects.

For multivariate analysis, the age, gender and BMI were adjusted using MaAsLin. Enterobacteriaceae was an abundant family significantly related to the fibrosis severity in the non-obese subjects (*p*=0.0108, *q*=0.214) (FIG. 2e to 2h). In phylum Firmicutes, Veillonellaceae showed a steep increase of the relative abundance in the non-obese subjects than the obese subjects (non-obese, p=0.0002, q=0.0195), but the abundance of Ruminococcaceae showed an inverse correlation with the fibrosis severity in the non-obese subjects (*p*=0.0019, *q*=0.0908). A representative genus of Ruminococcaceae, Ruminococcus also showed a significant inverse correlation according to the fibrosis severity (*p*=0.0009, *q*=0.135) (FIG. 10a to 10c). In addition, Veillonellaceae and Enterobacteriaceae showed a significant positive correlation with the serum free fatty acid (FFA) level in the non-obese subjects (*q*=0.178, *q*=0.118, respectively), but it did not in the obese subjects (FIG. 9a to 9e).

Adipo-IR and glycosylated hemoglobin (HbA1c) also showed a positive correlation according to the abundance of Veillonellaceae (adipo-IR, *q*=0.142; HbA1c, *q*=0.157). On the contrary, the serum FFA level showed an inverse correlation with the abundance of Ruminococcus in all subjects (*q*=0.0838) and non-obese subjects (*q*=0.0838), but it did not in the obese subjects (*q*=1.00).

In order to elucidate whether these remarkable microbiome changes in the non-obese subjects are related to the host gene effect, the association between bacteria and genetic mutations of *PNPL3, TM6SF2, MBOAT7-TMC4,* and *SREBF-2* using MaAsLin was analyzed. However, significant association of the four genetic mutations with three bacteria was not observed. Only actinomyces enriched the minor allele of TM6SF2 (C/T) (*q*=0.169) in the non-obese subjects (FIG. 11).

In addition to the three variables of age, gender and BMI, the presence of type 2 diabetes mellitus (DM) was well known to affect the general changes in the microbiome (Qin J, Li Y, Cai Z, Li S, Zhu J, Zhang F, et al. A metagenome-wide association study of gut microbiota in type 2 diabetes. Nature 2012;490:55-60.). After additional adjustment for DM, it was found that Enterobacteriaceae (*p*=0.00197, *q*=0.0616) and Faecalibacterium (*p*=0.00242, q=0.0707) were related to the presence of DM in all the subjects (FIG. 12a to 12d). In the non-obese subjects, not only depletion of Lachnospira (*p*=5.26×10⁻⁴, *q*=0.0676), but also the proliferation of Klebsiella (*p*=0.00339, *q*=0.141) belonging to Enterobacteriaceae in the obese subjects were also observed in the DM subjects.

In order to understand the interaction between the microbial components and gut microbiota network characteristics in the obese and non-obese subjects, co-expression of the taxa related to the fibrosis severity was measured, and the relative abundance was shown (FIG. 2i to 2k).

As a result, in the non-obese subjects, Veillonellaceae and Enterobacteriaceae had an inverse correlation with Ruminococcaceae (rho=-0.275 and -0.333, respectively), and Prevotellaceae showed an inverse correlation with Bacteroidaceae (rho=-0.391). However, the strong interaction between Veillonellaceae/Enterobacteriaceae and Ruminococcaceae was not observed in the obese and all subjects. In particular, the correlation of Veillonellaceae and fibrosis severity was not significant in the obese subjects and all subjects, and this suggests its specific role in progression of fibrosis in the non-obese subjects.

In summary, the proliferation of the specific taxa according to the fibrosis severity was more pronounced in the non-obese group than in the obese group.

### 4) Observation of fecal metabolite level according to fibrosis severity of non-obese and obese NAFLD subjects

The non-obese and obese NAFLD subjects had different fecal metabolite levels according to the fibrosis severity. Specifically, fecal metabolites mainly related to the gut microbiota were evaluated.

The composition of the total bile acid pool between the non-obese and obese subjects was various, and the non-obese subjects had an increased primary bile acid level according to the increased fibrosis stage (FIG. 3a).

The total fecal bile acid level was 3 times higher in the non-obese subjects having significant fibrosis (F2-4) than the subjects without fibrosis (F0) (FIG. 3b to FIG. 3g). In particular, the cholic acid (CA), chenodeoxycholic acid (CDCA), and ursodeoxycholic acid (UDCA) levels were increased according to the fibrosis severity increased in the non-obese subjects (FIG. 3b to FIG. 3g and FIG. 13a to 13e). The lithocholic acid (LCA) and deoxycholic acid (DCA) levels were significantly high in the obese subjects having significant fibrosis, and only lithocholic acid showed significant improvement after percentage display.

Among three SCFA, the fecal propionate level was gradually increased as fibrosis became severe in the non-obese subjects (non-obese; *p*=0.0032, obese; *p*=0.7979), and showed a significantly positive correlation with the amount of Veillonellaceae known as propionate-producing bacteria (*p*=0.0155) (FIG. 3h to 3j).

On the contrary to the bile acid profile, the correlation between the significant change of fecal SCFA and its bacterial taxa was observed only in the non-obese subjects (FIG. 4b). Ruminococcus (*p*=0.0189), Oscillospira (*p*=1.57×10⁻⁴) and Desulfovibrio (*p*=9.76×10⁻⁴) well-known as SCFA-producing bacteria showed an inverse correlation with the fecal propionate level. The change in the fecal butyrate level according to the fibrosis severity was not found in all the non-obese and obese subjects, and the reduction of Ruminococcaceae did not affect the fecal butyrate level (non-obese, *p*=0.597; obese, *p*=0.109).

### 5) Observation of bacterial taxa-metabolite network pattern in non-obese and obese NAFLD

A bacterial taxa-metabolite network showed a unique pattern in the non-obese and obese NAFLD. Specifically, when comparing the gut microbiota elements according to the fibrosis severity and obesity status, a clear change in the microbiome was observed only in the non-obese subjects. To investigate its core cause, NAFLD-associated genetic variant and intestinal metabolite analysis was performed.

Based on the result, co-expression of the taxa and metabolites was evaluated, and the interaction network was shown in FIG. 4. Strong interaction between bile acids was observed in all the non-obese and obese subjects. However, the bacterial taxa and metabolite co-expression pattern according to the fibrosis severity was different in the non-obese subjects and obese subjects: The non-obese subjects showed a more significant co-expression pattern than the obese subjects.

Interestingly, primary bile acid had an inverse correlation with Ruminococcaceae and Rikenellaceae known as indexes of healthy intestine in all the non-obese and obese subjects. Veillonellaceae exhibited a positive correlation with propionate, as well as primary bile acid. Bile acid usually has the potential to regulate growth of susceptible bacteria or to propagate relatively resistant bacteria regardless of obesity status. Nevertheless, the correlation of the intestinal bacterial taxa and fecal metabolites with the sever fibrosis was more remarkable in the non-obese NAFLD subjects than the obese subjects.

### 6) NAFLD prediction of non-obese subjects by microbiota and metabolite combination

The microbiota-metabolite combination accurately predicted significant fibrosis in the non-obese NAFLD subjects. Specifically, in order to evaluate the usefulness as a fibrosis-predicting biomarker of the gut microbiota and related fecal metabolites, AUROC for predicting significant fibrosis was compared (FIG. 5a and FIG. 5b).

Enterobacteriaceae, Veillonellaceae, and Ruminococcaceae were selected as most representative, and significant fibrosis-related bacterial taxa. As shown in FIG. 5a, the combined bacterial marker to predict significant fibrosis yielded an AUROC of 0.824 in the non-obese subjects (0.661 for all subjects; 0.584 for obese subjects).

In addition, Megamonas belonging to Veillonellaceae family and Ruminococcus belonging to Ruminococcaceae were selected. As shown in FIG. 5b, AUROC to predict significant fibrosis was yielded as 0.718 (0.673 for all subjects; 0.648 for obese subjects).

As fibrosis-related metabolites, four fecal metabolites (cholic acid, chenodeoxycholic acid, ursodeoxycholic acid, and propionate) were selected, and the combination of the four metabolites predicted significant fibrosis as AUROC of 0.758 in the non-obese subjects (0.505 for all subjects; 0.520 for obese subjects).

In case of addition of the intestinal metabolites to the bacterial marker at a family level, as shown in FIG. 5a, the predicting ability was significantly enhanced as improved AUROC of 0.977 (0.786 for all subjects; 0.609 for obese subjects). In addition, in case of addition of the intestinal metabolites to the bacterial marker at a genus level, as shown in FIG. 5b, it was enhanced as improved AUROC of 0.955 (0.590 for all subjects; 0.636 for obese subjects). The predictive ability of the novel microbiota-metabolite biomarker was significantly higher than FIB-4 widely used as a non-invasive biomarker of NAFLD.

The result demonstrated that the diagnosis accuracy of the combination of the identified intestinal bacterial taxa and fecal metabolite, for predicting significant fibrosis in NAFLD subjects was significantly higher in the non-obese subjects than the obese subjects, and clear differences of specific bacterial taxa and large intestine metabolite between the obese and non-obese NAFLD groups could be confirmed. This result emphasizes not only the importance of the gut microbiome as a risk factor explaining the pathogenesis of non-obese NAFLD, but also the importance of application in diagnosis of the novel microbiome-metabolite combination as a non-invasive biomarker for significant fibrosis in non-obese NAFLD.

## Claims

1. A kit for predicting or diagnosing a degree of risk of nonalcoholic fatty liver disease comprising a detection means detecting one or more detection markers selected from the group consisting of
(a) one or more detection markers selected from the group consisting of microbial biomarkers of nonalcoholic fatty liver disease;
(b) one or more detection markers selected from the group consisting of total bile acid and components of bile acid; and
(c) intestinal short chain fatty acid.

2. The kit according to claim 1, wherein the (a) microbial biomarkers of nonalcoholic fatty liver disease are one or more selected from the group consisting of Enterobacteriaceae, Veillonellaceae, Rikenellaceae, Fusobacteriaceae, Ruminococcaceae, Lachnospiraceae, Actinomycetaceae, Desulfovibrioceae and Desulfovibrionaceae.

3. The kit according to claim 1, wherein the (a) microbial biomarkers of nonalcoholic fatty liver disease are one or more selected from the group consisting of Citrobacter, Klebsiella, Veillonella, Megamonas, Fusobacterium, Ruminococcus, Faecalibacterium, Oscillospira, Coprococcus, Lachnospira, Actinomyces and Desulfovibrio.

4. The kit according to claim 1, wherein the (b) one or more detection markers selected from the group consisting of total bile acid and components of bile acid are one or more selected from the group consisting of total bile acid, cholic acid, chenodeoxycholic acid, ursodeoxycholic acid, lithocholic acid and deoxycholic acid.

5. The kit according to claim 1, wherein the (c) intestinal short chain fatty acid is one or more selected from the group consisting of acetate, propionate and butyrate.

6. The kit according to claim 1, wherein the kit comprises one or more selected from combinations of (a) to (h) below:
(a) one or more detection means capable of detecting one or more selected from the group consisting of Enterobacteriaceae, Veillonellaceae, Lachnospiraceae and Ruminococcaceae, respectively;
(b) one or more detection means capable of detecting one or more selected from the group consisting of total bile acid, cholic acid, chenodeoxycholic acid, ursodeoxycholic acid, lithocholic acid and deoxycholic acid, respectively;
(c) one or more detection means capable of detecting one or more selected from the group consisting of short chain fatty acid, acetate, propionate and butyrate, respectively;
(d) one or more detection means capable of detecting one or more selected from the group consisting of Enterobacteriaceae, Veillonellaceae, Lachnospiraceae, Ruminococcaceae, cholic acid, chenodeoxycholic acid, ursodeoxycholic acid and propionate, respectively;
(e) one or more detection means capable of detecting one or more selected from the group consisting of Ruminococcus, Faecalibacterium, Oscillospira, Coprococcus, Lachnospira, total bile acid, cholic acid, chenodeoxycholic acid, ursodeoxycholic acid, lithocholic acid and deoxycholic acid, respectively;
(f) one or more detection means capable of detecting one or more selected from the group consisting of Ruminococcus, Faecalibacterium, Oscillospira, Coprococcus, Lachnospira and fecal propionate, respectively;
(g) one or more detection means capable of detecting one or more selected from the group consisting of Veillonella, Megamonas, total bile acid, cholic acid, chenodeoxycholic acid, ursodeoxycholic acid, lithocholic acid and deoxycholic acid, respectively; and
(h) one or more detection means capable of detecting one or more selected from the group consisting of Veillonella, Megamonas and fecal propionate, respectively.

7. The kit according to claim 1, wherein the kit comprises one or more selected from combinations of (i) to (k) below:
(i) one or more detection means capable of detecting one or more selected from the group consisting of Enterobacteriaceae, Veillonellaceae, Ruminococcaceae, Citrobacter, Klebsiella, Veillonella, Megamonas, Ruminococcus, Faecalibacterium and Oscillospira, respectively;
(j) one or more detection means capable of detecting one or more selected from the group consisting of cholic acid, chenodeoxycholic acid, ursodeoxycholic acid and metabolites thereof, respectively; and
(k) one or more detection means capable of detecting one or more selected from the group consisting of intestinal short chain fatty acid and propionate, respectively.

8. The kit according to claim 1, wherein the kit comprises a detection means capable of detecting Enterobacteriaceae, Veillonellaceae, and Ruminococcaceae.

9. The kit according to claim 1, wherein the kit comprises a detection means capable of detecting *Megamonas* and *Ruminococcus.*

10. The kit according to claim 1, wherein the kit comprises a detection means capable of detecting cholic acid, chenodeoxycholic acid, ursodeoxycholic acid, and propionate.

11. The kit according to claim 8 or claim 9, wherein the kit comprises a detection means capable of detecting cholic acid, chenodeoxycholic acid, ursodeoxycholic acid, and propionate.

12. The kit according to claim 1, wherein the nonalcoholic fatty liver disease is nonalcoholic fatty liver, nonalcoholic steatohepatitis, or cirrhosis.

13. The kit according to claim 1, wherein the nonalcoholic fatty liver disease is non-obese nonalcoholic fatty liver disease.

14. The kit according to claim 1, wherein the predicting the degree of risk is predicting the severity of fibrosis comprising F=0 to F=4.

15. The kit according to claim 1, wherein the kit is for a non-obese patient.

16. A method for providing information for predicting or diagnosing a degree of risk of nonalcoholic fatty liver disease comprising detecting one or more detection markers selected from the group consisting of
(a) one or more detection markers selected from the group consisting of microbial biomarkers of nonalcoholic fatty liver disease;
(b) one or more detection markers selected from the group consisting of total bile acid and components of bile acid; and
(c) intestinal short chain fatty acid.

17. The method according to claim 16, wherein the (a) microbial biomarkers of nonalcoholic fatty liver disease are one or more selected from the group consisting of Enterobacteriaceae, Veillonellaceae, Rikenellaceae, Fusobacteriaceae, Ruminococcaceae, Lachnospiraceae, Actinomycetaceae, Desulfovibrioceae and Desulfovibrionaceae.

18. The method according to claim 16, wherein the (a) microbial biomarkers of nonalcoholic fatty liver disease are one or more selected from the group consisting of *Citrobacter, Klebsiella, Veillonella, Megamonas, Fusobacterium, Ruminococcus, Faecalibacterium, Oscillospira, Coprococcus, Lachnospira, Actinomyces,* and *Desulfovibrio.*

19. The method according to claim 16, wherein the (b) one or more detection markers selected from the group consisting of total bile acid and components of bile acid are one or more selected from the group consisting of total bile acid, cholic acid, chenodeoxycholic acid, ursodeoxycholic acid, lithocholic acid and deoxycholic acid.

20. The method according to claim 16, wherein the (c) intestinal short chain fatty acid is one or more selected from the group consisting of acetate, propionate and butyrate.

21. The method according to claim 16, wherein the method comprises one or more steps selected from combinations of (i) to (k) below:
(i) comparing abundances of one or more selected from the group consisting of Enterobacteriaceae, Veillonellaceae, Ruminococcaceae, Citrobacter, Klebsiella, Veillonella, Megamonas, Ruminococcus, Faecalibacterium and Oscillospira measured in a subject, with a reference value of a healthy individual;
(j) comparing the contents in feces of one or more selected from the group consisting of cholic acid, chenodeoxycholic acid, ursodeoxycholic acid and metabolites thereof measured in a subject, with a reference value of a healthy individual; and
(k) comparing the contents in feces of one or more selected from the group consisting of intestinal short chain fatty acid and propionate measured in a subject, with a reference value of a healthy individual.

22. The method according to claim 16, wherein the method comprises detecting cholic acid, chenodeoxycholic acid, ursodeoxycholic acid, and propionate.

23. The method according to claim 16, wherein the method comprises detecting Enterobacteriaceae, Veillonellaceae, and Ruminococcaceae.

24. The method according to claim 16, wherein the method comprises detecting *Megamonas* and *Ruminococcus.*

25. The method according to claim 23 or claim 24, wherein the method further comprises detecting cholic acid, chenodeoxycholic acid, ursodeoxycholic acid, and propionate.

26. The method according to claim 16, wherein the nonalcoholic fatty liver disease is nonalcoholic fatty liver, nonalcoholic steatohepatitis, or cirrhosis.

27. The method according to claim 16, wherein the nonalcoholic fatty liver disease is non-obese nonalcoholic fatty liver disease.

28. The method according to claim 16, wherein the method is for a non-obese patient.

29. A method for screening a therapeutic agent for nonalcoholic fatty liver disease, comprising
(1) administering a test substance to an experimental animal having nonalcoholic fatty liver disease;
(2) measuring one or more of detection markers selected from the group consisting of (a) one or more detection markers selected from the group consisting of microbial biomarkers of nonalcoholic fatty liver disease; (b) one or more detection markers selected from the group consisting of total bile acid and components of bile acid; and (c) one or more detection markers selected from the group consisting of intestinal short chain fatty acids, in an experimental animal untreated with the test substance and the experimental animal administered with the test substance; and
(3) comparing the measured results in a control group untreated with the test substance and the experimental animal administered with the test substance.

30. The method according to claim 29, wherein the (a) microbial biomarkers of nonalcoholic fatty liver disease are one or more selected from the group consisting of Enterobacteriaceae, Veillonellaceae, Rikenellaceae, Fusobacteriaceae, Ruminococcaceae, Lachnospiraceae, Actinomycetaceae, Desulfovibrioceae and Desulfovibrionaceae.

31. The method according to claim 29, wherein the (a) microbial biomarkers of nonalcoholic fatty liver disease are one or more selected from the group consisting of *Citrobacter, Klebsiella, Veillonella, Megamonas, Fusobacterium, Ruminococcus, Faecalibacterium, Oscillospira, Coprococcus, Lachnospira, Actinomyces,* and *Desulfovibrio.*

32. The method according to claim 29, wherein the (b) one or more detection markers selected from the group consisting of total bile acid and components of bile acid are total bile acid, cholic acid, chenodeoxycholic acid, ursodeoxycholic acid, lithocholic acid, and deoxycholic acid.

33. The method according to claim 29, wherein the (c) intestinal short chain fatty acid is one or more selected from the group consisting of acetate, propionate and butyrate.

34. The method for screening a therapeutic agent of nonalcoholic fatty liver according to claim 25, wherein the method comprises one or more steps selected from combinations of (i) to (p) below:
(i) comparing abundances of one or more selected from the group consisting of Enterobacteriaceae, Veillonellaceae, Ruminococcaceae, Citrobacter, Klebsiella, Veillonella, Megamonas, Ruminococcus, Faecalibacterium and Oscillospira, measured before and after administration of a candidate substance of therapeutic agent;
(j) comparing the contents in feces of one or more selected from the group consisting of cholic acid, chenodeoxycholic acid, ursodeoxycholic acid and metabolites thereof, measured before and after administration of a candidate substance of therapeutic agent;
(k) comparing the contents in feces of one or more selected from the group consisting of intestinal short chain fatty acids and propionate, measured before and after administration of a candidate substance of therapeutic agent;
(1) comparing abundances of Enterobacteriaceae, Veillonellaceae, and Ruminococcaceae, measured before and after administration of a candidate substance of therapeutic agent;
(m) comparing abundances of *Megamonas* and *Ruminococcus,* measured before and after administration of a candidate substance of therapeutic agent;
(n) comparing the contents in feces of cholic acid, chenodeoxycholic acid, ursodeoxycholic acid and propionate, measured before and after administration of a candidate substance of therapeutic agent;
(o) comprising the (1) and (n); and
(p) comprising the (m) and (n).

35. The method for screening a therapeutic agent for nonalcoholic fatty liver disease according to claim 29, wherein the nonalcoholic fatty liver disease is nonalcoholic fatty liver, nonalcoholic steatohepatitis or cirrhosis.

36. The method for screening a therapeutic agent for nonalcoholic fatty liver disease according to claim 29, wherein the nonalcoholic fatty liver disease is non-obese nonalcoholic fatty liver disease.

37. The method according to claim 29, wherein the detection marker comprises cholic acid, chenodeoxycholic acid, ursodeoxycholic acid, and propionate.

38. The method according to claim 29, wherein the detection marker comprises Enterobacteriaceae, Veillonellaceae, and Ruminococcaceae.

39. The method according to claim 29, wherein the detection marker comprises *Megamonas* and *Ruminococcus.*

40. The method according to claim 38 or claim 39, wherein the detection marker further comprising detecting cholic acid, chenodeoxycholic acid, ursodeoxycholic acid, and propionate.

41. A method for treatment of nonalcoholic fatty liver disease, comprising
predicting or diagnosing a degree of risk of nonalcoholic fatty liver disease of a subject using the kit according to any one of claim 1 to claim 15; and
administering a pharmaceutical composition or a food composition for improving or treating nonalcoholic fatty liver to the subject, when the subject is determined to have the risk of nonalcoholic fatty liver disease.

42. The method according to any one of claim 16 to claim 28, wherein the method further comprises administering a pharmaceutical composition or a food composition for improving or treating nonalcoholic fatty liver.
